# EUROPÄISCHE PATENTSCHRIFT

(11) **EP 1 812 430 B1**
(45) Veröffentlichungstag und Bekanntmachung des Hinweises auf die Patenterteilung: **15.04.2009**
(21) Anmeldenummer: 05776232.0
(22) Anmeldetag: 30.08.2005
(51) Int. Cl.: C07D 417/12, A61K 31/4439, A61P 9/12

(54) **SUBSTITUIERTE PHENYLAMINOTHIAZOLE UND IHRE VERWENDUNG**
SUBSTITUTED PHENYLAMINOTHIAZOLES AND USE THEREOF
PHENYLAMINOTHIAZOLES SUBSTITUES, ET LEUR UTILISATION

(30) Priorität: 03.09.2004 DE 102004042607
(43) Veröffentlichungstag der Anmeldung: 01.08.2007
(73) Patentinhaber: Bayer HealthCare AG, 51368 Leverkusen (DE)
(72) Erfinder: ERGÜDEN, Jens-Kerim, 42489 Wülfrath (DE); KARIG, Gunter, 51069 Köln (DE); ROSENTRETER, Ulrich, 42349 Wuppertal (DE); ALBRECHT, Barbara, 42489 Wülfrath (DE); HENNINGER, Kerstin, 42115 Wuppertal (DE); HÜTTER, Joachim, 42349 Wuppertal (DE); DIEDRICHS, Nicole, 42553 Velbert (DE); NELL, Peter, 42115 Wuppertal (DE); ARNDT, Sabine, 44227 Dortmund (DE); HÜBSCH, Walter, 42113 Wuppertal (DE); KNORR, Andreas, 40699 Erkrath (DE); SCHLEMMER, Karl-Heinz, 42113 Wuppertal (DE); BROHM, Dirk, 40822 Mettmann (DE)
(86) Internationale Anmeldenummer: PCT/EP2005/009316
(87) Internationale Veröffentlichungsnummer: WO 2006/027142

(56) Entgegenhaltungen:
- WO-A-03/053441

## Beschreibung

Die vorliegende Anmeldung betrifft neue Phenylaminothiazol-Derivate, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, vorzugsweise zur Behandlung und/oder Prävention von Hypertonie und anderen kardiovaskulären Erkrankungen.

Adenosin, ein Purin-Nukleosid, ist in allen Zellen vorhanden und wird unter einer Vielzahl von physiologischen und pathophysiologischen Stimuli freigesetzt. Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf den Blutdruck, die Herzfrequenz, auf die Ausschüttung von Neurotransmittern und auf die Lymphozyten-Differenzierung.

Diese Wirkungen von Adenosin zielen darauf ab, das Sauerstoffangebot der betroffenen Organe zu erhöhen bzw. den Stoffwechsel dieser Organe zu drosseln, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Als "Adenosinrezeptor-selektive Liganden" werden erfindungsgemäß solche Substanzen bezeichnet, die selektiv an einen oder mehrere Subtypen der Adenosin-rezeptoren binden und dabei entweder die Wirkung des Adenosin nachahmen (Adenosin-Agonisten) oder dessen Wirkung blockieren (Adenosin-Antagonisten) können.

Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die A1- oder A3-Rezeptoren über eine Hemmung der Adenylatzyklase eine Abnahme des intrazellulären cAMP-Gehalts bewirkt.

Im Herz-Kreislaufsystem sind die Hauptwirkungen der Aktivierung von Adenosin-Rezeptoren: Bradykardie, negative Inotropie und Protektion des Herzens vor Ischämie ("preconditioning") über A1-Rezeptoren, Dilation der Gefäße über A2a- und A2b-Rezeptoren sowie Inhibition der Fibroblasten und Glattmuskelzellproliferation über A2b-Rezeptoren.

Im Falle von A1-Agonisten (Kopplung bevorzugt über Gᵢ-Proteine) wird dabei eine Abnahme des intrazellulären cAMP-Gehaltes beobachtet (bevorzugt nach direkter Vorstimulation der Adenylatzyklase durch Forskolin). Entsprechend führen A2a- und A2b-Agonisten (Kopplung bevorzugt über Gₛ-Proteine) zu einer Zunahme und A2a- und A2b-Antagonisten zu einer Abnahme im cAMP-Gehalt der Zellen. Im Falle der A2-Rezeptoren ist eine direkte Vorstimulation der Adenylatzyklase durch Forskolin nicht hilfreich.

Die Aktivierung von A2b-Rezeptoren durch Adenosin oder spezifische A2b-Agonisten führt über die Erweiterung von Gefäßen zu einer Blutdrucksenkung. Die Blutdrucksenkung ist von einem reflektorischen Herzfrequenzanstieg begleitet. Der Herzfrequenzanstieg kann durch die Aktivierung von A1-Rezeptoren durch spezifische A1-Agonisten reduziert werden.

Die kombinierte Wirkung von selektiven A1/A2b-Agonisten auf das Gefäßsystem und die Herzfrequenz resultiert somit in einer systemischen Blutdrucksenkung ohne relevanten Herzfrequenzanstieg. Mit einem solchen pharmakologischen Profil könnten duale A1/A2b-Agonisten zur Behandlung z.B. der Hypertonie beim Menschen eingesetzt werden.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M. E. Olah, H. Ren, J. Ostrowski, K. A. Jacobson, G. L. Stiles, "Cloning, expression, and characterization of the unique bovine A1 adenosine receptor. Studies on the ligand binding site by site-directed mutagenesis" in J. Biol. Chem. 267 (1992), Seiten 10764-10770, deren Offenbarung hiermit im vollen Umfang durch Bezugnahme eingeschlossen ist).

Die Wirkung der Substanzen an solchen Zelllinien lässt sich erfassen durch biochemische Messung des intrazellulären Botenstoffes cAMP (siehe hierzu die Druckschrift K. N. Klotz, J. Hessling, J. Hegler, C. Owman, B. Kull, B. B. Fredholm, M. J. Lohse, "Comparative pharmacology of human adenosine receptor subtypes - characterization of stably transfected receptors in CHO cells" in Naunyn Schmiedebergs Arch. Pharmacol. 357 (1998), Seiten 1-9, deren Offenbarung hiermit im vollen Umfang durch Bezugnahme eingeschlossen ist).

Bei den aus dem Stand der Technik bekannten, als "Adenosinrezeptor-spezifisch", geltenden

Liganden handelt es sich überwiegend um Derivate auf Basis des natürlichen Adenosins [S.-A. Poulsen und R. J. Quinn, "Adenosine receptors: new opportunities for future drugs" in Bioorganic and Medicinal Chemistry 6 (1998), Seiten 619-641]. Diese aus dem Stand der Technik bekannten Adenosin-Liganden haben jedoch meistens den Nachteil, dass sie nicht wirklich rezeptorspezifisch wirken, schwächer wirksam sind als das natürliche Adenosin oder nach oraler Applikation nur sehr schwach wirksam sind. Deshalb werden sie überwiegend nur für experimentelle Zwecke verwendet.

WO 02/06237 offenbart arylsubstituierte Dicyanopyridine als calciumabhängige Kaliumkanalöffner und ihre Verwendung bei der Behandlung von Erkrankungen des Urogenitaltrakts. Weiterhin werden in WO 01/25210 und WO 02/070485 substituierte 2-Thio-3,5-dicyano-4-aryl-6-aminopyridine als Adenosin-Rezeptorliganden für die Behandlung von Erkrankungen beschrieben. In WO 03/053441 werden spezifisch substituierte 2-Thio-3,5-dicyano-4-phenyl-6-aminopyridine als selektive Liganden des Adenosin-A1-Rezeptors für die Behandlung insbesondere kardiovaskulärer Erkrankungen offenbart. In WO 02/50071 werden Aminothiazol-Derivate als Tyrosin-Kinase-Inhibitoren für die Behandlung verschiedener Krankheiten beschrieben.

Aufgabe der vorliegenden Erfindung ist daher die Bereitstellung neuer Verbindungen, die als selektive, duale Agonisten des Adenosin-A1- und A2b-Rezeptors wirken und als solche zur Behandlung und/oder Prävention insbesondere von Hypertonie und anderen kardiovaskulären Erkrankungen geeignet sind.

Gegenstand der vorliegenden Erfindung sind Verbindungen der Formel (I) 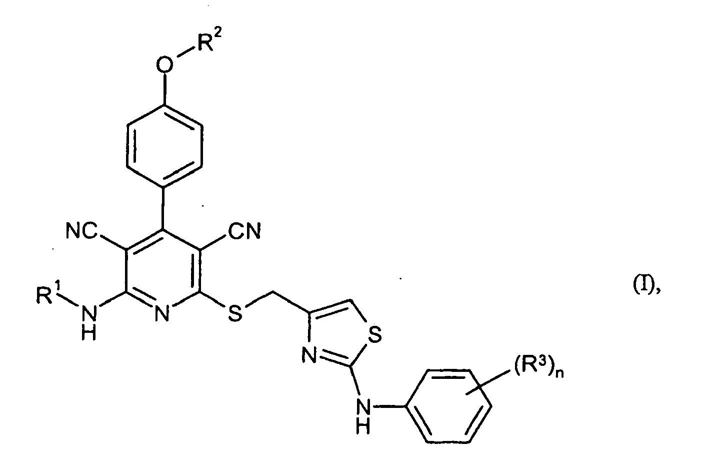 in welcher
- R¹: für wasserstoff oder für (C₁-C₆)-Alkyl steht, das durch Hydroxy, Amino, Mono- oder Di-(C₁-C₄)-alkylamino, Pyrrolidino, Piperidino, Morpholino, Piperazino oder *N*'-Methylpiperazino substituiert sein kann,
- R²: für (C₂-C₆)-Alkyl steht, das ein- oder zweifach, gleich oder verschieden, mit Substituenten ausgewählt aus der Gruppe von Hydroxy, (C₁-C₄)-Alkoxy, Amino, Mono- und Di-(C₁-C₄)-alkylamino substituiert ist,
- R³: für einen Substituenten ausgewählt aus der Gruppe von Halogen, Cyano, Nitro, (C₁-C₆)-Alkyl, Hydroxy, (C₁-C₆)-Alkoxy, Amino, Mono- und Di-(C₁-C₆)-alkylamino, Carboxyl und (C₁-C₆)-Alkoxycarbonyl steht,
worin Alkyl und Alkoxy ihrerseits jeweils bis zu fünffach mit Fluor substituiert sein können,
und
- n: für die Zahl 0, 1, 2, 3, 4 oder 5 steht,
wobei für den Fall, dass der Substituent R³ mehrfach auftritt, seine Bedeutungen gleich oder verschieden sein können,
sowie ihre Salze, Solvate und Solvate der Salze.

Erfindungsgemäße Verbindungen sind die Verbindungen der Formel (I) und deren Salze, Sölvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl, (C₂-C₆)-Alkyl, (C₁-C₄)-Alkyl und (C₂-C₄)-Alkyl stehen im Rahmen der Erfindung für einen geradkettigen oder verzweigten Alkylrest mit 1 bis 6, 2 bis 6, 1 bis 4 bzw. 2 bis 4 Kohlenstoffatomen. Bevorzugt ist ein geradkettiger oder verzweigter Alkylrest mit 1 bis 4 bzw. 2 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl und n-Hexyl.
(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy und tert.-Butoxy.
(C₁-C₆)-Alkoxycarbonyl und (C₁-C₄)-Alkoxycarbonyl stehen im Rahmen der Erfindung für einen geradkettigen oder verzweigten Alkoxyrest mit 1 bis 6 bzw. 1 bis 4 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Mono-(C₁-C₆)-Alkylamino und Mono-(C₁-C₄)-Alkylamino stehen im Rahmen der Erfindung für eine Amino-Gruppe mit einem geradkettigen oder verzweigten Alkylsubstituenten, der 1 bis 6 bzw. 1 bis 4 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamine, Ethylamino, n-Propylamino, Isopropylamino und tert.-Butylamino.
Di-(C₁-C₆)-Alkylamino und Di-(C₁-C₄)-Alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N,N*-Dimethylamino, *N,N*-Diethylamino, *N*-Ethyl-*N*-methylamino, *N*-Methyl-*N*-n-propylamino, *N*-Isopropyl-*N*-n-p-ropylamino, *N*-tert.-Butyl-*N*-methylamino, *N*-Ethyl-*N*-n-pentylamino und *N*-n-Hexyl-*N*-methylamino.
Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem oder zwei gleichen oder verschiedenen Substituenten.

Bevorzugt im Rahmen der vorliegenden Erfindung sind Verbindungen der Formel (I),
in welcher
- R¹: für Wasserstoff oder für (C₁-C₄)-Alkyl steht, das durch Hydroxy, Amino oder Dimethylamino substituiert sein kann,
- R²: für (C₂-C₄)-Alkyl steht, das ein- oder zweifach, gleich oder verschieden, mit Substituenten ausgewählt aus der Gruppe von Hydroxy, Methoxy und Amino substituiert ist,
- R³: für einen Substituenten ausgewählt aus der Gruppe von Halogen, Cyano, Nitro, (C₁-C₄)-Alkyl, Hydroxy, (C₁-C₄)-Alkoxy, Amino, Mono- und Di-(C₁-C₄)-alkylamino, Carboxyl und (C₁-C₄)-Alkoxycarbonyl steht,
worin Alkyl und Alkoxy ihrerseits jeweils bis zu dreifach mit Fluor substituiert sein können,
und
- n: für die Zahl 0, 1 oder 2 steht,
wobei für den Fall, dass der Substituent R³ zweifach auftritt, seine Bedeutungen gleich oder verschieden sein können,
sowie ihre Salze, Solvate und Solvate der Salze.

Besonders bevorzugt im Rahmen der vorliegenden Erfindung sind Verbindungen der Formel (I),
in welcher
- R¹: für Wasserstoff steht,
- R²: für Ethyl, n-Propyl oder iso-Propyl steht, die jeweils ein- oder zweifach, gleich oder verschieden, mit Substituenten ausgewählt aus der Gruppe von Hydroxy, Methoxy und Amino substituiert sind,
- R³: für einen Substituenten ausgewählt aus der Gruppe von Fluor, Chlor, Brom, Cyano, Nitro, Methyl, Ethyl, Trifluormethyl, Hydroxy, Methoxy, Ethoxy, Amino, Mono- und Dimethylamino, Carboxyl, Methoxycarbonyl und Ethoxycarbonyl steht,
und
- n: für die Zahl 0, 1 oder 2 steht,
wobei für den Fall, dass der Substituent R³ zweifach auftritt, seine Bedeutungen gleich oder verschieden sein können,
sowie ihre Salze, Solvate und Solvate der Salze.

Weiterer Gegenstand der vorliegenden Erfindung ist ein Verfahren zur Herstellung der erfindungsgemäßen Verbindungen der Formel (I),
**dadurch gekennzeichnet, dass** man Verbindungen der Formel (II) 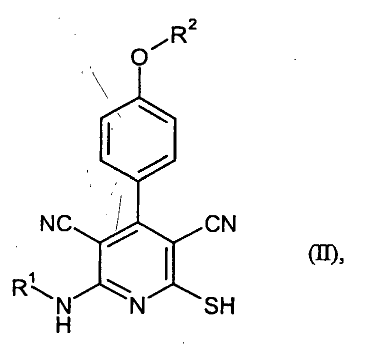 worin R¹ und R² jeweils die zuvor angegebenen Bedeutungen haben,
in einem inerten Lösungsmittel in Gegenwart einer Base mit einer Verbindung der Formel (III) 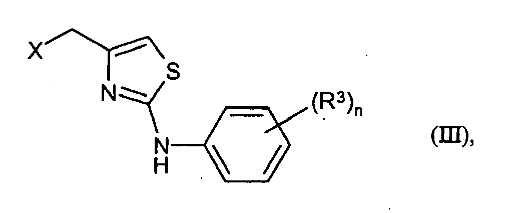 worin R³ und n jeweils die zuvor angegebenen Bedeutungen haben und
- X: für eine geeignete Abgangsgruppe, vorzugsweise für Halogen, insbesondere Chlor, Brom oder Iod, oder für Mesylat, Tosylat oder Triflat steht,
umsetzt
und die Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

Das zuvor beschriebene Verfahren kann durch folgendes Formelschema beispielhaft erläutert werden:

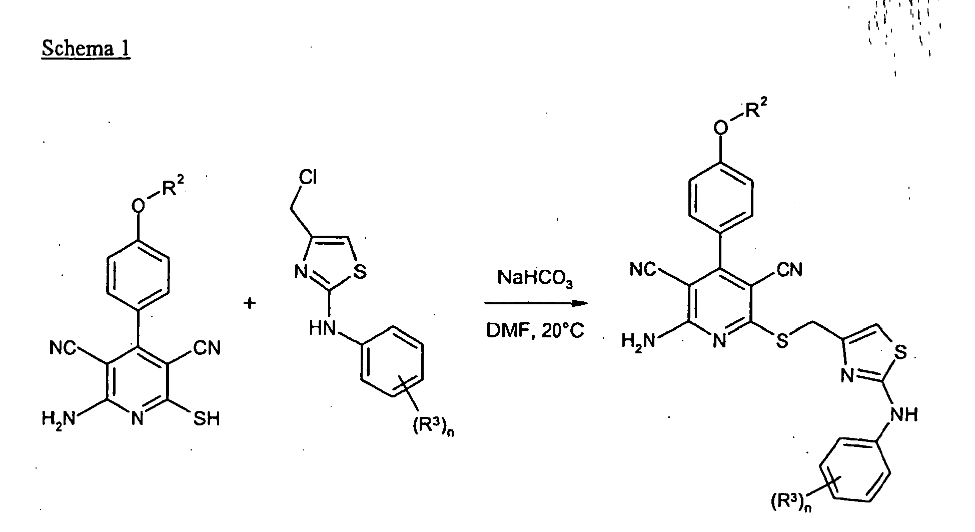

Als Lösungsmittel für das erfindungsgemäße Verfahren eignen sich alle organischen Lösungsmittel, die unter den Reaktionsbedingungen inert sind. Hierzu gehören Alkohole wie Methanol, Ethanol und Isopropanol, Ketone wie Aceton und Methylethylketon, acyclische und cyclische Ether wie Diethylether, Tetrahydrofuran und Dioxan, Ester wie Essigsäureethylester oder Essigsäurebutylester, Kohlenwasserstoffe wie Benzol, Toluol, Xylol, Hexan oder Cyclohexan, chlorierte Kohlenwasserstoffe wie Dichlormethan oder Chlorbenzol, oder andere Lösungsmittel wie Dimethylformamid, Acetonitril, Pyridin oder Dimethylsulfoxid. Wasser ist als Lösungsmittel ebenfalls geeignet. Ebenso ist es möglich, Gemische der zuvor genannten Lösungsmittel einzusetzen. Bevorzugt als Lösungsmittel ist Dimethylformamid.

Als Basen eignen sich die üblichen anorganischen oder organischen Basen. Hierzu gehören bevorzugt Alkalihydroxide wie beispielsweise Natrium- oder Kaliumhydroxid, Alkalicarbonate wie Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder Amide wie Natriumamid, Lithium-bis-(trimethylsilyl)-amid oder Lithiumdiisopropylamid, oder metallorganische Verbindungen wie Butyllithium oder Phenyllithium, oder organische Amine wie Triethylamin, Pyridin, 1,8-Diazabicyclo[5.4.0]undec-7-en (DBU) oder 1,5-Diazablcyclo[4.3.0]non-5-en (DBN). Bevorzugt sind Alkalicarbonate und -hydrogencarbonate.

Die Base kann hierbei in einer Menge von 1 bis 10 Mol, bevorzugt von 1 bis 5 Mol, insbesondere von 1 bis 4 Mol, bezogen auf 1 Mol der Verbindung der Formel (II), eingesetzt werden.

Die Reaktion erfolgt im Allgemeinen in einem Temperaturbereich von -78°C bis +140°C, bevorzugt im Bereich von -20°C bis +60°C, insbesondere bei 0°C bis +40°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Verbindungen der Formel (II), worin R¹ für Wasserstoff steht, sind dem Fachmann an sich bekannt oder nach üblichen, literaturbekannten Methoden herstellbar. Insbesondere kann auf die folgenden Druckschriften verwiesen werden, deren jeweiliger Inhalt durch Bezugnahme eingeschlossen wird:
a) Dyachenko et al., Russian Journal of Chemistry 33 (7), 1014-1017 (1997) und 34 (4), 557-563 (1998);
b) Dyachenko et al., Chemistry of Heterocyclic Compounds 34 (2), 188-194 (1998);
c) Qintela et al., European Journal of Medicinal Chemistry 33, 887-897 (1998);
d) Kandeel et al., Zeitschrift für Naturforschung 42b, 107-111 (1987).

Die Verbindungen der Formel (II), worin R¹ für Wasserstoff steht, können auch ausgehend von Verbindungen der Formel (IV) 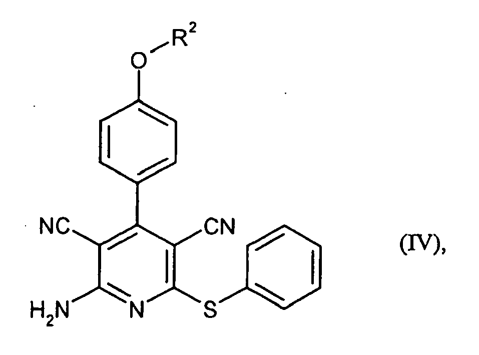 worin R² die zuvor angegebene Bedeutung hat,
durch Umsetzung mit einem Alkalisulfid hergestellt werden. Diese Herstellungsmethode kann durch folgendes Formelschema beispielhaft erläutert werden:

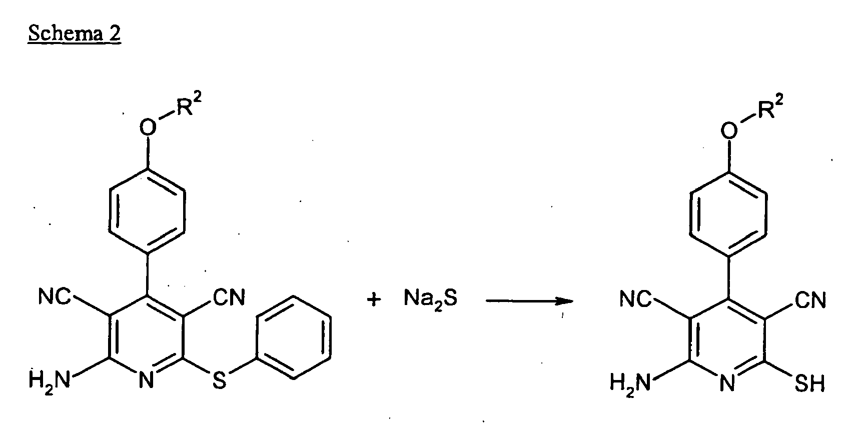

Als Alkalisulfid wird vorzugsweise Natriumsulfid in einer Menge von 1 bis 10 Mol, bevorzugt von 1 bis 5 Mol, insbesondere von 1 bis 4 Mol, bezogen auf 1 Mol der Verbindung der Formel (IV), eingesetzt.

Als Lösungsmittel geeignet sind alle organischen Lösungsmittel, die unter den Reaktionsbedingungen inert sind. Hierzu gehören bevorzugt *N,N*-Dimethylformamid, *N*-Methylpyrrolidinon, Pyridin und Acetonitril. Ebenso ist es möglich, Gemische der zuvor genannten Lösungsmittel einzusetzen. Besonders bevorzugt ist *N,N*-Dimethylformamid.

Die Reaktion erfolgt im Allgemeinen in einem Temperaturbereich von +20°C bis +140°C, bevorzugt im Bereich von +20°C bis +120°C, insbesondere bei +60°C bis +100°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Verbindungen der Formel (IV) können analog zu den in den folgenden Druckschriften beschriebenen Verbindungen hergestellt werden:
a) Kambe et al., Synthesis, 531-533 (1981);
b) Elnagdi et al., Z. Naturforsch. 47b, 572-578 (1991).

Verbindungen der Formel (II), worin R¹ nicht für Wasserstoff steht, können hergestellt werden, indem man Verbindungen der Formel (IV) zunächst mit Kupfer(II)chlorid und Isoamylnitrit in einem geeigneten Lösungsmittel in Verbindungen der Formel (V) 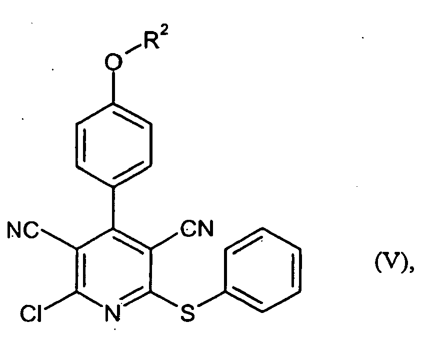 worin R² die zuvor angegebene Bedeutung hat,
überführt, diese anschließend mit einer Verbindung der Formel (VI)

R^{1A}-NH₂ (VI),

worin
- R^{1A}: die oben angegebene Bedeutung von R¹ hat, jedoch nicht für Wasserstoff steht,
zu Verbindungen der Formel (VII) 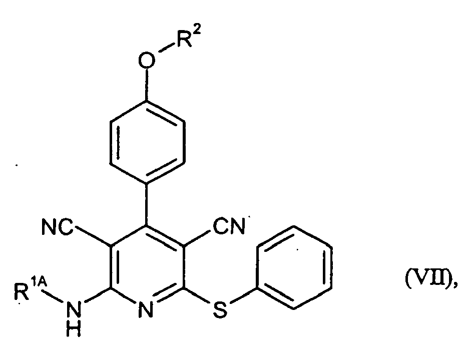 worin R^{1A} und R² jeweils die zuvor angegebenen Bedeutungen haben,
umsetzt und abschließend mit Natriumsulfid in Verbindungen der Formel (II) überführt.

Das zuvor beschriebene Verfahren kann durch das folgende Formelschema beispielhaft erläutert werden:

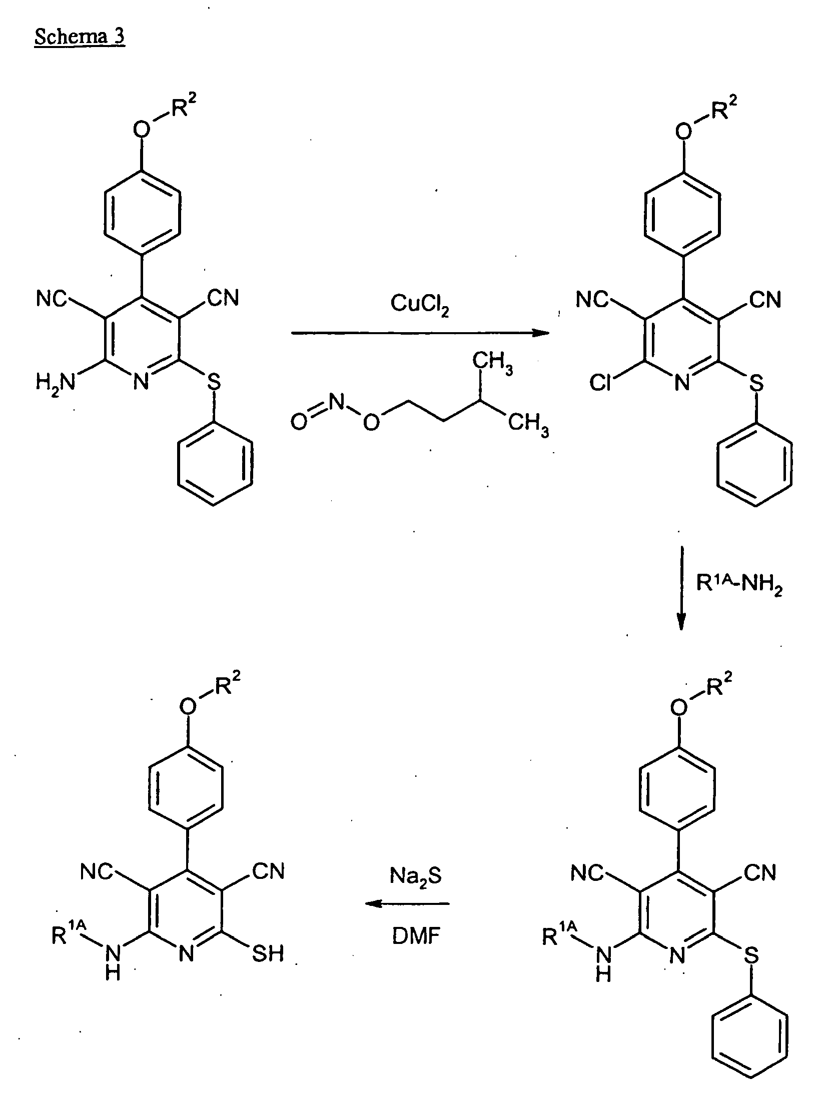

Der Verfahrensschritt (IV) → (V) erfolgt im Allgemeinen mit einem Molverhältnis von 2 bis 12 Mol Kupfer(II)chlorid und 2 bis 12 Mol Isoamylnitrit bezogen auf 1 Mol der Verbindung der Formel (IV).

Als Lösungsmittel für diesen Verfahrensschritt eignen sich alle organischen Lösungsmittel, die unter den Reaktionsbedingungen inert sind. Hierzu gehören acyclische und cyclische Ether wie Diethylether und Tetrahydrofuran, Ester wie Essigsäureethylester oder Essigsäurebutylester, Kohlenwasserstoffe wie Benzol, Toluol, Xylol, Hexan oder Cyclohexan, chlorierte Kohlenwasserstoffe wie Dichlormethan, Dichlorethan oder Chlorbenzol, oder andere Lösungsmittel wie Dimethylformamid, Acetonitril oder Pyridin. Ebenso ist es möglich, Gemische der zuvor genannten Lösungsmittel einzusetzen. Bevorzugte Lösungsmittel sind Acetonitril und Dimethylformamid.

Die Reaktion erfolgt im Allgemeinen in einem Temperaturbereich von -78°C bis +180°C, bevorzugt im Bereich von +20°C bis +100°C, insbesondere bei +20°C bis +60°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (V) + (VI) → (VII) erfolgt im Allgemeinen mit einem Molverhältnis von 1 bis 8 Mol der Verbindung der Formel (VI) bezogen auf 1 Mol der Verbindung der Formel (V).

Als Lösungsmittel für diesen Verfahrensschritt eignen sich alle organischen Lösungsmittel, die unter den Reaktionsbedingungen inert sind. Hierzu gehören Alkohole wie Methanol, Ethanol und Isopropanol, Ketone wie Aceton und Methylethylketon, acyclische und cyclische Ether wie Diethylether und Tetrahydrofuran, Ester wie Essigsäureethylester oder Essigsäurebutylester, Kohlenwasserstoffe wie Benzol, Toluol, Xylol, Hexan oder Cyclohexan, chlorierte Kohlenwasserstoffe wie Dichlormethan, Dichlorethan oder Chlorbenzol, oder andere Lösungsmittel wie Dimethylformamid, Acetonitril, Pyridin oder Dimethylsulfoxid. Wasser ist als Lösungsmittel ebenfalls geeignet. Ebenso ist es möglich, Gemische der zuvor genannten Lösungsmittel einzusetzen. Bevorzugtes Lösungsmittel ist Dimethylformamid.

Die Reaktion erfolgt im Allgemeinen in einem Temperaturbereich von -78°C bis +180°C, bevorzugt im Bereich von +20°C bis +160°C, insbesondere bei +20 bis +40°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (VII) → (II) erfolgt im Allgemeinen mit einem Molverhältnis von 1 bis 8 Mol Natriumsulfid bezogen auf 1 Mol der Verbindung der Formel (VII).

Als Lösungsmittel für diesen Verfahrensschritt eignen sich alle organischen Lösungsmittel, die unter den Reaktionsbedingungen inert sind. Hierzu gehören Alkohole wie Methanol, Ethanol und Isopropanol, Ketone wie Aceton und Methylethylketon, acyclische und cyclische Ether wie Diethylether und Tetrahydrofuran, Ester wie Essigsäureethylester oder Essigsäurebutylester, Kohlenwasserstoffe wie Benzol, Toluol, Xylol, Hexan oder Cyclohexan, chlorierte Kohlenwasserstoffe wie Dichlormethan, Dichlorethan oder Chlorbenzol, oder andere Lösungsmittel wie Dimethylformamid, Acetonitril, Pyridin oder Dimethylsulfoxid. Ebenso ist es möglich, Gemische der zuvor genannten Lösungsmittel einzusetzen. Bevorzugtes Lösungsmittel ist Dimethylformamid.

Die Reaktion erfolgt im Allgemeinen in einem Temperaturbereich von -78°C bis +180°C, bevorzugt im Bereich von +20°C bis +160°C, insbesondere bei +40°C bis +100°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Verbindungen der Formel (VI) sind entweder kommerziell erhältlich, dem Fachmann bekannt oder nach üblichen Methoden herstellbar.

Verbindungen der Formel (III) können aus Verbindungen der Formel (VIII) 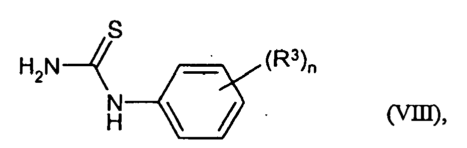 worin R³ und n die zuvor angegebenen Bedeutungen haben,
durch Umsetzung mit einem 1,3-Dihalogenaceton hergestellt werden. Diese Herstellungsmethode kann durch folgendes Formelschema beispielhaft erläutert werden:

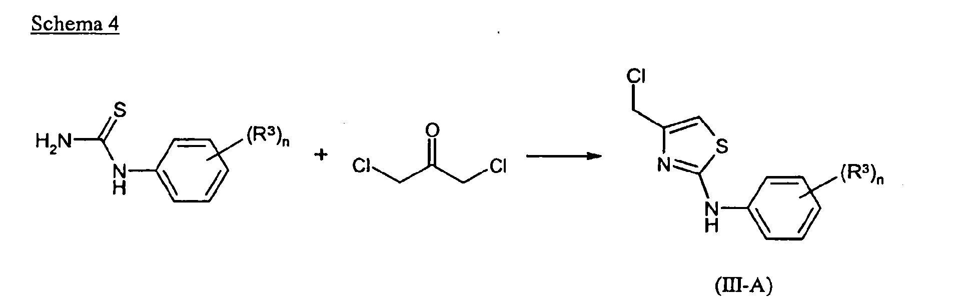

Die Verbindungen der Formel (III-A) können dabei entweder analog zur Literatur [I. Simiti et al., Chem. Ber. 95, 2672-2679 (1962)] hergestellt und isoliert werden oder sie können *in situ* erzeugt und direkt weiter mit einer Verbindung der Formel (II) umgesetzt werden. Bevorzugt ist die *in situ-*Erzeugung aus 1,3-Dichloraceton und einer Verbindung der Formel (VIII) in Dimethylformamid oder Ethanol. Die Herstellung erfolgt im Allgemeinen in einem Temperaturbereich von 0°C bis +140°C, bevorzugt im Bereich von +20°C bis +120°C, insbesondere bei +80°C bis +100°C.

Die Verbindungen der Formel (VIII) sind entweder kommerziell erhältlich, dem Fachmann bekannt oder nach üblichen Methoden herstellbar.

Überraschenderweise zeigen die erfindungsgemäßen Verbindungen ein nicht vorhersehbares, wertvolles pharmakologisches Wirkspektrum und sind daher insbesondere zur Prophylaxe und/oder Behandlung von Erkrankungen geeignet.

Die pharmazeutische Wirksamkeit der erfindungsgemäßen Verbindungen lässt sich durch ihre Wirkung als selektive Liganden an Adenosin-A1- und A2b-Rezeptoren erklären. Sie wirken hierbei als duale A1/A2b-Agonisten.

Als "selektive Liganden an Adenosin-A1- und A2b-Rezeptoren" werden im Rahmen der vorliegenden Erfindung solche Adenosin-Rezeptorliganden bezeichnet, bei denen einerseits eine deutliche Wirkung an A1- und A2b-Adenosin-Rezeptor-Subtypen und andererseits keine oder eine deutliche schwächere Wirkung (Faktor 10 oder höher) an A2a- und A3-Adenosin-Rezeptor-Subtypen zu beobachten ist, wobei bezüglich der Testmethoden für die Wirk-Selektivität Bezug genommen wird auf die im Abschnitt B-1. beschriebenen Tests.

Die Verbindungen der Formel (I) sind allein oder in Kombination mit einem oder mehreren anderen Wirkstoffen zur Prophylaxe und/oder Behandlung verschiedener Erkrankungen geeignet, so beispielsweise insbesondere bei Hypertonie und anderen Erkrankungen des Herzkreislauf-Systems (kardiovaskulären Erkrankungen). Geeignete Kombinationswirkstoffe sind insbesondere Wirkstoffe zur Behandlung von Hypertonie und/oder koronaren Herzkrankheiten wie Betablocker, Calcium-Antagonisten, Diuretika, ACE-Hemmer, AT1-Antagonisten und Nitrate.

Im Sinne der vorliegenden Erfindung sind unter Erkrankungen des Herzkreislauf-Systems bzw. kardiovaskulären Erkrankungen beispielsweise insbesondere neben der Hypertonie die folgenden Erkrankungen zu verstehen: Koronare Restenose wie z.B. Restenose nach Ballondilatation von peripheren Blutgefäßen, Tachykardien, Arrhythmien, periphere und kardiale Gefäßerkrankungen, stabile und instabile Angina pectoris, Vorhof- und Kammerflimmern und Herzinsuffizienz.

Weiterhin eignen sich die Verbindungen der Formel (I) beispielsweise insbesondere auch zur Reduktion des von einem Infarkt betroffenen Myokardbereichs sowie zur Prophylaxe von Sekundärinfarkten.

Des weiteren eignen sich die Verbindungen der Formel (I) beispielsweise insbesondere zur Prophylaxe und/oder Behandlung von thromboembolischen Erkrankungen und Ischämien wie Myokardinfarkt, Hirnschlag und transitorischen ischämischen Attacken.

Weitere Indikationsgebiete, für die sich die Verbindungen der Formel (I) eignen, sind beispielsweise insbesondere die Prophylaxe und/oder Behandlung von Erkrankungen des Urogenitalbereiches, wie z.B. Reizblase, erektile Dysfunktion und weibliche sexuelle Dysfunktion, daneben aber auch die Prophylaxe und/oder Behandlung von inflammatorischen Erkrankungen, wie z.B. Asthma und entzündliche Dermatosen, von neuroinflammatorischen Erkrankungen des Zentralnervensystems, wie beispielsweise Zustände nach Hirninfarkt, der Alzheimer-Erkrankung, weiterhin auch von neurodegenerativen Erkrankungen sowie von Schmerzzuständen, Krebs und Übelkeit und Erbrechen in Verbindung mit Krebstherapien.

Ein weiteres Indikationsgebiet sind beispielsweise insbesondere die Prophylaxe und/oder Behandlung von Erkrankungen der Atemwege wie beispielsweise Asthma, chronische Bronchitis, Lungenemphysem, Bronchiektasien, zystische Fibrose (Mukoviszidose) und pulmonale Hypertonie.

Schließlich kommen die Verbindungen der Formel (I) beispielsweise insbesondere auch für die Prophylaxe und/oder Behandlung von Diabetes, insbesondere Diabetes mellitus, des metabolischen Syndroms sowie von Dyslipidämien in Betracht.

Die vorliegende Erfindung betrifft auch die Verwendung der Verbindungen der Formel (I) zur Herstellung von Arzneimitteln zur Prophylaxe und/oder Behandlung der zuvor genannten Krankheitsbilder.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfmdungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Verwendete Abkürzungen:

| | |
|---|---|
| Bsp. | Beispiel |
| DC | Dünnschichtchromatographie |
| DCI | direkte chemische Ionisation (bei MS) |
| DMF | *N*,*N*-Dimethylformamid |
| DMSO | Dimethylsulfoxid |
| d. Th. | der Theorie (bei Ausbeute) |
| EE | Ethylacetat (Essigsäureethylester) |
| EI | Elektronenstoß-Ionisation (bei MS) |
| ESI | Etektrospray-Ionisation (bei MS) |
| Fp. | Schmelzpunkt |
| ges. | gesättigt |
| h | Stunde(n) |
| HPLC | Hochdruck-, Hochleistungsflüssigchromatographie |
| konz. | konzentriert |
| LC-MS | Flüssigchromatographie-gekoppelte Massenspektroskopie |
| LDA | Lithiumdiisopropylamid |
| Lit. | Literatur(stelle) |
| Lsg. | Lösung |
| min | Minute(n) |
| MS | Massenspektroskopie |
| NMR | Kernresonanzspektroskopie |
| RP-HPLC | reverse phase HPLC |
| RT | Raumtemperatur |
| Rₜ | Retentionszeit (bei HPLC) |
| THF | Tetrahydrofuran |
| verd. | verdünnt |
| wäßr. | wässrig |

### HPLC- und LC-MS-Methoden:

### Methode 1 (HPLC):

Instrument: Hewlett Packard Series 1050; Säule: Symmetry TM C18 3.9 x 150 mm; Flüss: 1.5 ml/min; Eluent A: Wasser, Eluent B: Acetonitril; Gradient: → 0.6 min 10% B → 3.8 min 100% B → 5.0 min 100% B → 5.5 min 10% B; Stopzeit: 6.0 min; Injektionsvolumen: 10 µl; Diodenarraydetektor-Signal: 214 und 254 nm.

### Methode 2 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 3 (LC-MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 4 (LC-MS):

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 5 (LC-MS):

Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%-ige Ameisensäure / 1, Eluent B: Acetonitril + 500 µl 50%-ige Ameisensäure / l; Gradient: 0.0 min 10% B → 7.0 min 95% B → 9.0 min 95% B; Ofen: 35°C; Fluss: 0.0 min 1.0 ml/min → 7.0 min 2.0 ml/min → 9.0 min 2.0 ml/min; UV-Detektion: 210 nm.

### Methode 6 (HPLC):

Instrument: HP 1100 mit DAD-Detektion; Säule: Kromasil RP-18, 60 mm x 2 mm, 3.5 µm; Eluent A: 5 ml HClO₄ / l Wasser, Eluent B: Acetonitril; Gradient: 0 min 2% B → 0.5 min 2% B → 4.5 min 90% B → 9 min 90% B; Fluss: 0.75 ml/min; Ofen: 30°C; UV-Detektion: 210 nm.

### Methode 7 (HPLC):

Instrument: HP 1100 mit DAD-Detektion; Säule: Kromasil RP-18, 60 mm x 2 mm, 3.5 µm; Eluent A: 5 ml HClO₄ / l Wasser, Eluent B: Acetonitril; Gradient: 0 min 2% B → 0.5 min 2% B → 4.5 min 90% B → 6.5 min 90% B; Fluss: 0.75 ml/min; Ofen: 30°C; UV-Detektion: 210 nm.

### Ausgangsverbindungen und Intermediate:

### Beispiel 1A

### 4-[(2,2-Dimethyl-1,3-dioxolan-4-yl)methoxy]benzaldehyd

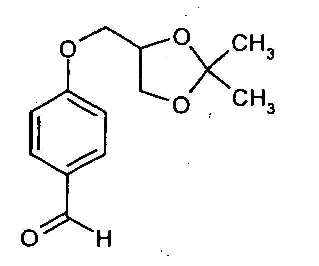

Zu einer Lösung von 13.2 g (100 mmol) 1,2-*O*-Isopropylidenglycerin in 250 ml trockenem THF werden 39.3 g (150 mmol) Triphenylphosphin gegeben und der Ansatz 30 min bei RT gerührt. Man kühlt auf ca. 0°C ab und gibt 12.2 g (100 mmol) 4-Hydroxybenzaldehyd und 31.9 g (150 mmol) Azodicarbonsäurediisopropylester (DIAD) hinzu. Die gelbe Reaktionslösung wird für 16 h bei RT gerührt. Anschließend wird der Ansatz am Rotationsverdampfer eingeengt und der Rückstand auf 150 ml ges. Natriumhydrogencarbonat-Lsg. gegeben. Man extrahiert mit Essigsäureethylester (dreimal je 150 ml) und trocknet die vereinigten organischen Phasen über Natriumsulfat. Nach Entfernen des Lösungsmittels am Rotationsverdampfer wird das Rohprodukt an Kieselgel 60 (Laufmittelgradient Cyclohexan → Cyclohexan/Essigsäureethylester 2:1) chromatographisch gereinigt.
Ausbeute: 5.03 g (21 % d. Th.)
LC-MS (Methode 3): Rₜ = 1.86 min; MS (ESIpos): m/z = 237 [M+H]⁺.

### Beispiel 2A

### {4-[(2,2-Dimethyl-1,3-dioxolan-4-yl)methoxy]benzyliden}malononitril

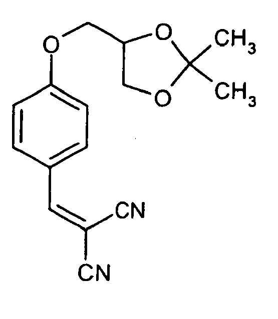

0.13 g (1.98 mmol) Malonsäuredinitril, 0.45 g (1.90 mmol) der Verbindung aus Beispiel 1A und 5.7 µl (0.06 mmol) Piperidin werden in Ethanol gelöst und der Ansatz für 3.5 h unter Rückfluss erhitzt. Die Reaktionslösung wird eingeengt und der Rückstand an Kieselgel 60 (Laufmittelgradient Cyclohexan → Cyclohexan/Essigsäureethylester 2:1) chromatographisch gereinigt.
Ausbeute: 0.43 g (79% d. Th.)
¹H-NMR (400 MHz, CDCl₃): δ = 7.91 (d, 2H), 7.65 (s, 1H), 7.03 (d, 2H), 4.51 (m, 1H) 4.19 (dd, 1H), 4.14 (dd, 1H), 4.06 (dd, 1H), 3.91 (dd, 1H), 1.46 (s, 3H), 1.41 (s, 3H).
MS (DCI, NH₃): m/z = 302 [M+NH₄]⁺.

### Beispiel 3A

### 2-Amino-4-{4-[(2,2-dimethyl-1,3-dioxolan-4-yl)methoxy]phenyl}-6-mercaptopyridin-3,5-dicarbonitril

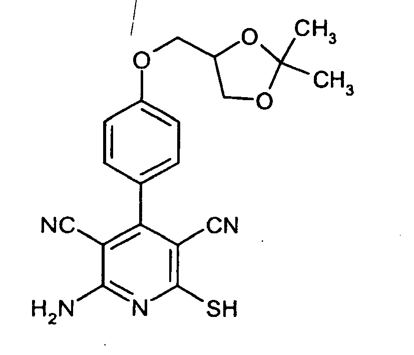

0.43 g (1.51 mmol) der Verbindung aus Beispiel 2A, 0.38 g (3.78 mmol) Cyanothioacetamid und 0.38 g (3.78 mmol) 4-Methylmorpholin werden in 15 ml Ethanol gelöst und der Ansatz für 6 h unter Rückfluss gerührt. Die Reaktionslösung wird nach dem Abkühlen am Rotationsverdampfer eingeengt und der Rückstand an Kieselgel 60 chromatographiert. Nach Abtrennung von Nebenkomponenten (Laufmittelgradient Cyclohexan → Cyclohexan/Essigsäureethylester 1:1) werden die Produktfraktionen eluiert (Laufmittelgradient Essigsäureethylester → Essigsäureethylester/Methanol 20:1). Anschließend erfolgt eine Feinreinigung über präparative HPLC (Säule: YMC GEL ODS-AQ S-5 / 15 µm; Laufmittelgradient: Acetonitril/Wasser 10:90 → 95:5).
Ausbeute: 88 mg (15% d. Th.)
¹H-NMR (400 MHz, DMSO-d₆): δ = 12.96 (br. s, 1H), 7.90 (br. s, 2H), 7.46 (d, 2H), 7.12 (d, 2H), 4.44 (m, 1H), 4.18-4.02 (m, 3H), 3.79 (m, 1H), 1.37 (s, 3H), 1.32 (s, 3H).
LC-MS (Methode 3): Rₜ = 1.76 min; MS (ESIpos): m/z = 383 [M+H]⁺.

### Beispiel 4A

### 4-[(4-{[(6-Amino-3,5-dicyano-4-{4-[(2,2-dimethyl-1,3-dioxolan-4-yl)methoxy]phenyl}pyridin-2-yl)thio]methyl}-1,3-thiazol-2-yl)amino]benzoesäure

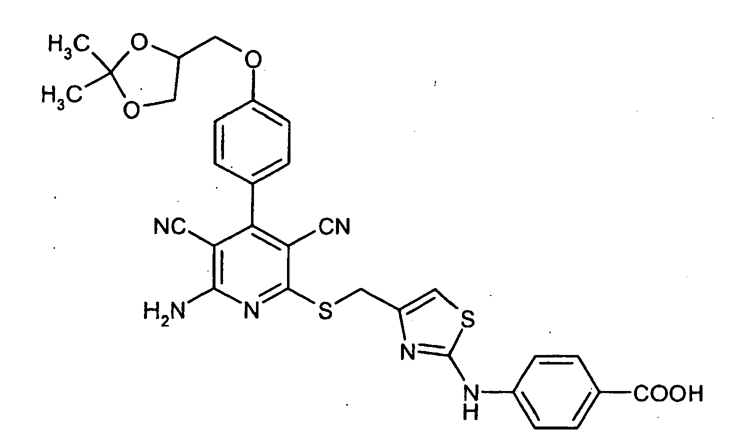

177 mg (0.90 mmol) 4-Carboxyphenylthioharnstoff und 111 mg (0.87 mmol) 1,3-Dichloraceton werden in 3 ml DMF gelöst und die Reaktionslösung 60 min bei 100°C gerührt. Nach dem Abkühlen werden 230 mg (0.60 mmol) der Verbindung aus Beispiel 3A und 151 mg (1.80 mmol) Natriumhydrogencarbonat zugegeben und der Ansatz weitere 16 h bei RT gerührt. Das Reaktionsgemisch wird direkt über präparative HPLC (Säule: YMC GEL ODS-AQ S-5 / 15 µm; Laufmittelgradient: Acetonitril/Wasser 10:90 → 95:5) chromatographisch gereinigt.
Ausbeute: 134 mg (36% d. Th.)
¹H-NMR (400 MHz, CDCl₃): δ = 12.5 (m, 1H), 10.6 (s, 1H), 8.07 (br. s, 2H), 7.86 (d, 2H), 7.67 (d, 2H), 7.49 (d, 2H), 7.12 (d, 2H), 7.07 (s, 1H), 4.50 (s, 2H), 4.44 (m, 1H), 4.16-4.03 (m, 3H), 3.78 (dd, 1H), 1.37 (s, 3H); 1.31 (s, 3H).
LC-MS (Methode 4): Rₜ = 2.51 min; MS (ESIpos): m/z = 615 [M+H]⁺.

### Beispiel 5A

### 2-Amino-4-{4-[(2,2-dimethyl-1,3-dioxolan-4-yl)methoxy]phenyl}-6-[({2-[(4-fluorphenyl)amino]-1,3-thiazol-4-yl}methyl)thio]pyridin-3,5-dicarbonitril

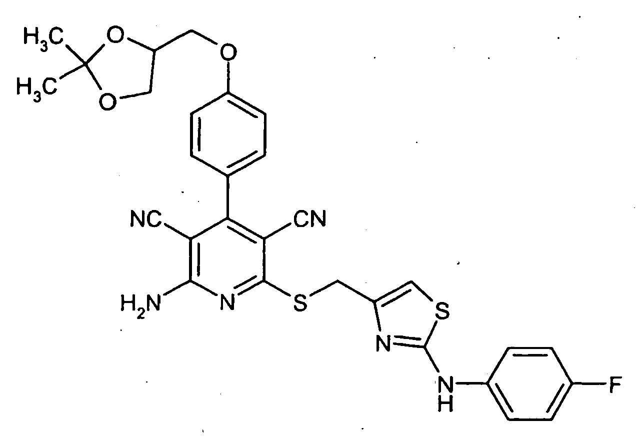

102 mg (0.6 mmol) 4-Fluorphenylthioharnstoff und 73.6 mg (0.58 mmol) 1,3-Dichloraceton werden in 2.5 ml Ethanol gelöst und der Ansatz 60 min unter Rückfluss gerührt. Man lässt abkühlen und engt am Rotationsverdampfer ein. Der Rückstand wird in 1.5 ml DMF aufgenommen, 153 mg (0.4 mmol) der Verbindung aus Beispiel 3A und 101 mg (1.2 mmol) Natriumhydrogencarbonat zugegeben und die Reaktionslösung weitere 16 h bei RT gerührt. Das Reaktionsgemisch wird direkt über präparative HPLC (Säule: YMC GEL ODS-AQ S-5 / 15 µm; Laufmittelgradient: Acetonitril/Wasser 10:90 → 95:5) chromtatograhisch gereinigt.
Ausbeute: 62 mg (26% d. Th.)
¹H-NMR (400 MHz, DMSO-d₆): δ = 10.24 (s, 1H), 8.08 (br. s, 2H), 7.62 (dd, 2H), 7.47 (d, 2H), 7.13 (m, 4H), 6.97 (s, 1H), 4.49-4.39 (m, 3H), 4.10 (m, 3H), 3.78 (dd, 1H), 1.36 (s, 3H), 1.31 (s, 3H).
LC-MS (Methode 2): Rₜ = 2.51 min; MS (ESIpos): m/z = 589 [M+H]⁺.

Die in Tabelle 1 aufgeführten Beispiele werden aus den entsprechenden Ausgangsverbindungen analog zu Beispiel 5A hergestellt:

**Tabelle 1**

| **Beispiel Nr.** | **Struktur** | **MS (ESI) [M+H]⁺** | **LC-MS Rₜ [min] (Methode)** | **Ausbeute (% d. Th.)** |
|---|---|---|---|---|
| **6A** | 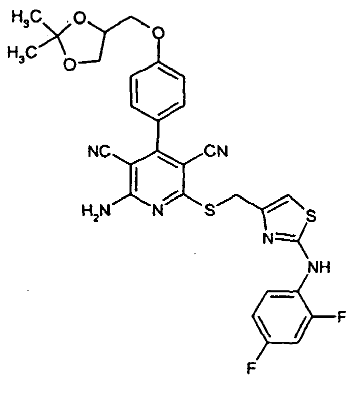 | 607 | 2.62 (3) | 39 |
| **7A** | 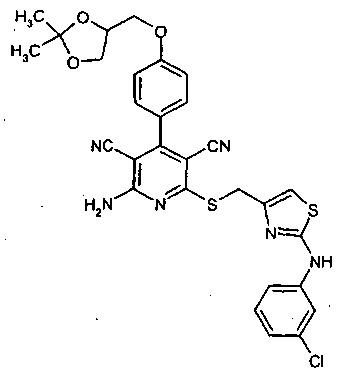 | 605 | 2.96 (4) | 53 |
| **8A** | 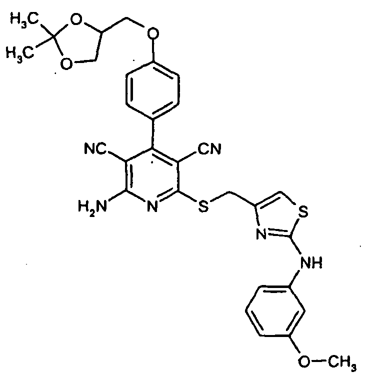 | 601 | 2.80 (4) | 40 |
| **9A** | 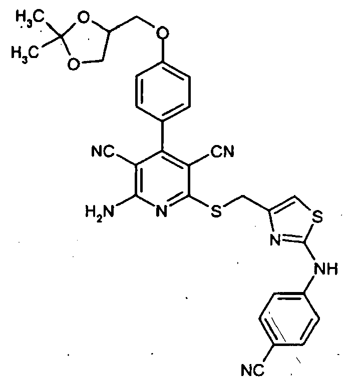 | 596 | 2.74 (4) | 38 |
| **10A** | 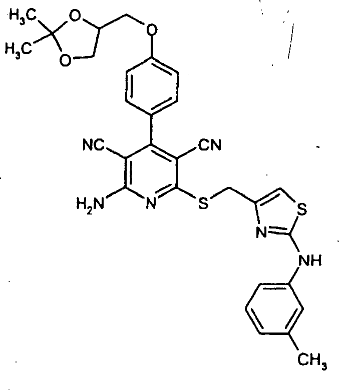 | 585 | 2.89 (4) | 46 |

### Beispiel 11A

### (S)-4-[(2,2-Dimethyl-1,3-dioxolan-4-yl)methoxy]benzaldehyd

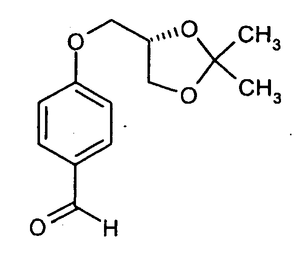

1.79 g (14.6 mmol) *p*-Hydroxybenzaldehyd werden in absolutem DMF (10 ml) gelöst und mit 14.2 g (102.5 mmol) Kaliumcarbonat und 3.3 g (22.0 mmol) (*R*)-(+)-4-Chlormethyl-2,2-dimethyl-1,3-dioxolan versetzt. Man erhitzt für 24 h auf 150°C. Anschließend wird der Ansatz am Rotationsverdampfer eingeengt und der Rückstand zwischen Dichlormethan und Wasser verteilt. Man extrahiert mit Dichlormethan (dreimal je 20 ml), wäscht mit ges. Kochsalz-Lösung und trocknet die vereinigten organischen Phasen über Magnesiumsulfat. Nach Entfernen des Lösungsmittels am Rotationsverdampfer wird das Rohprodukt an Kieselgel 60 (Laufmittel: Cyclohexan/Essigsäureethylester 5:1) chromatographisch gereinigt.
Ausbeute: 2.12 g (61 % d. Th.)
LC-MS (Methode 2): Rₜ = 1.97 min; MS (ESIpos): m/z = 237 [M+H]⁺.

### Beispiel 12A

### (S)-2-Amino-4-{4-[(2,2-dimethyl-1,3-dioxolan-4-yl)methoxy]phenyl}-6-mercaptopyridin-3,5-dicarbonitril

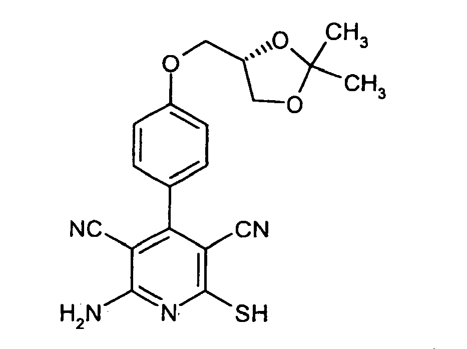

1.52 g (6.43 mmol) der Verbindung aus Beispiel 11A, 1.29 g (12.9 mmol) Cyanothioacetamid und 1.3 g (12.9 mmol) 4-Methylmorpholin werden in 15 ml Ethanol gelöst und der Ansatz für 3 h unter Rückfluss gerührt. Danach wird für 18 h bei RT gerührt. Die Reaktionslösung wird am Rotationsverdampfer eingeengt und der Rückstand an Kieselgel 60 chromatographiert (Laufmittel: Dichlormethan/Ethanol 10:1).
Ausbeute: 1.06 g (43% d. Th.)
LC-MS (Methode 3): Rₜ = 1.75 min; MS (ESIpos): m/z = 383 [M+H]⁺.

### Bespiel 13A

### (S)-2-Amino-4-{4-[(2,2-dimethyl-1,3-dioxolan-4-yl)methoxy]phenyl}-6-[({2-[(4-fluorphenyl)-amino]-1,3-thiazol-4-yl}methyl)thio]pyridin-3,5-dicarbonitril

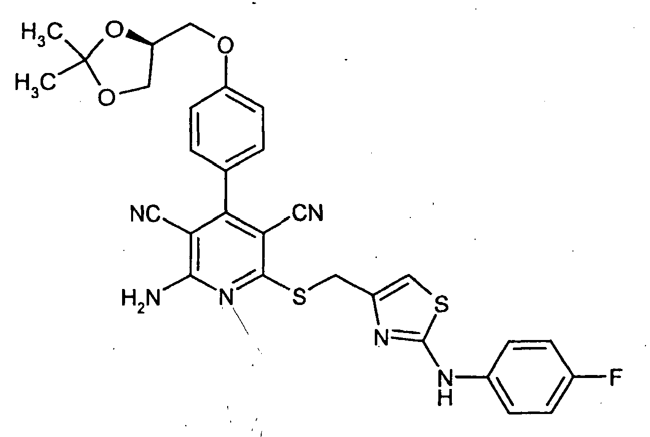

Die Synthese erfolgt analog zu Beispiel 5A ausgehend vom enantiomerenreinen Ausgangsmaterial aus Beispiel 12A.
Ausbeute: 47% d. Th.
LC-MS (Methode 3): Rₜ = 2.58 min; MS (ESIpos): m/z = 589 [M+H]⁺.

Die in Tabelle 2 aufgeführten Beispiele werden aus den entsprechenden Ausgangsverbindungen analog zu Beispiel 5A oder 13A bzw. des entsprechenden Enantiomers hergestellt:

**Tabelle 2**

| **Beispiel Nr.** | **Struktur** | **MS (ESI) [M+H]⁺** | **LC-MS Rₜ [min] (Methode)** | **Ausbeute (% d. Th.)** |
|---|---|---|---|---|
| **14A** | 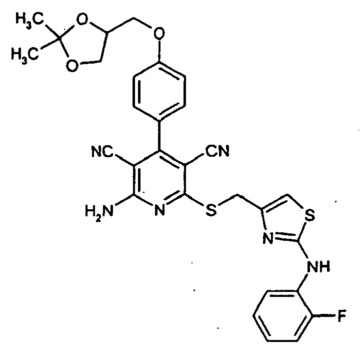 | 589 | 2.77 (2) | 50 |
| **15A** | 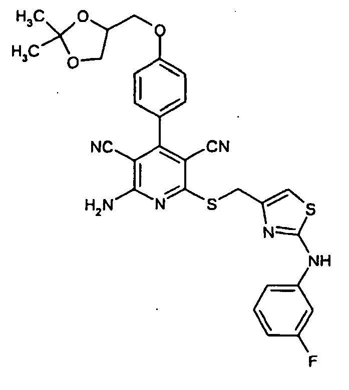 | 589 | 2.60 (3) | 36 |
| **16A** | 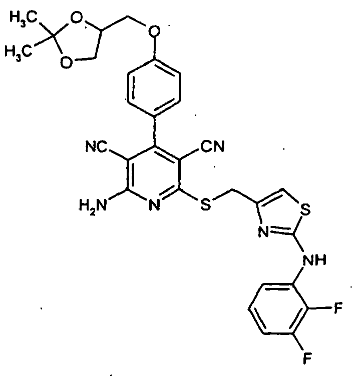 | 607 | 2.64 (3) | 52 |
| **17A** | 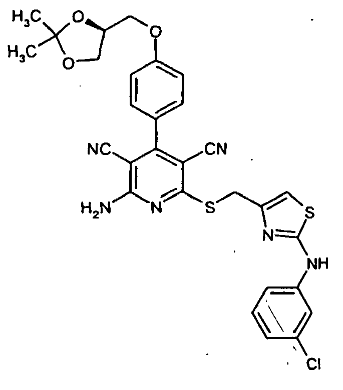 | 605 | 2.71 (3) | 33 |
| **18A** | 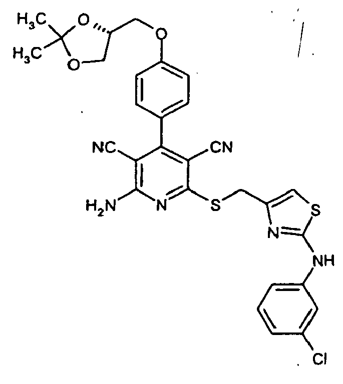 | 605 | 2.71 (3) | 54 |
| **19A** | 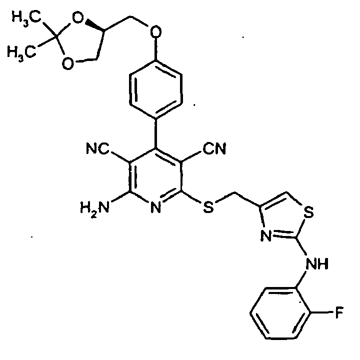 | 589 | 2.76 (2) | 61 |
| **20A** | 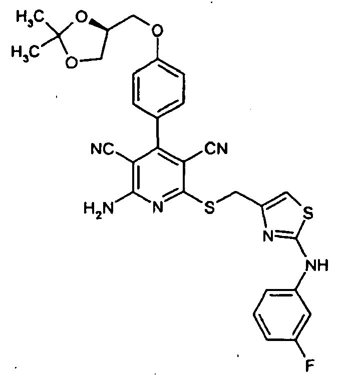 | 589 | 2.60 (3) | 18 |
| **21A** | 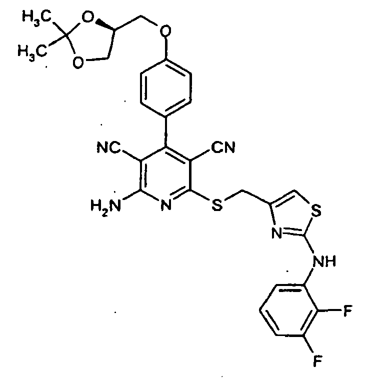 | 607 | 2.87 (4) | 41 |
| **22A** | 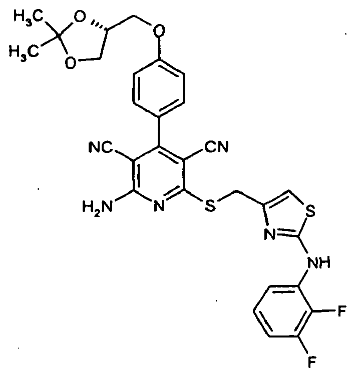 | 607 | 2.83 (2) | 23 |
| **23A** | 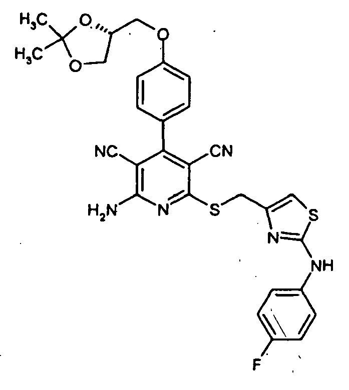 | 589 | 2.58 (3) | 80 |
| **24A** | 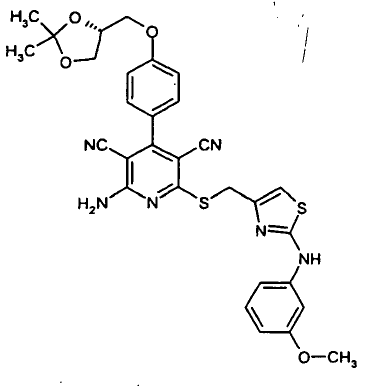 | 601 | 2.88 (4) | 67 |
| **25A** | 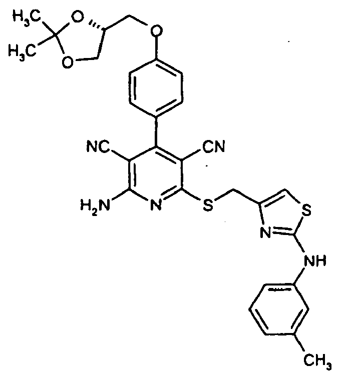 | 585 | 3.01(4) | 62 |
| **26A** | 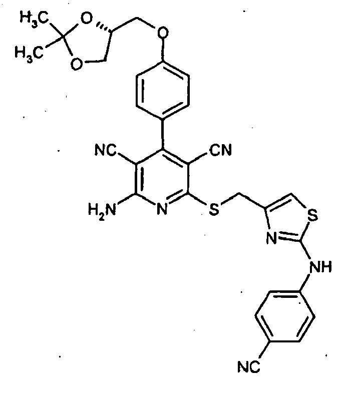 | 596 | 2.84 (4) | 45 |
| **27A** | 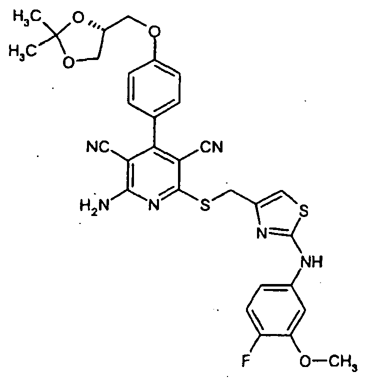 | 619 | 2.79 (4) | 68 |
| **28A** | 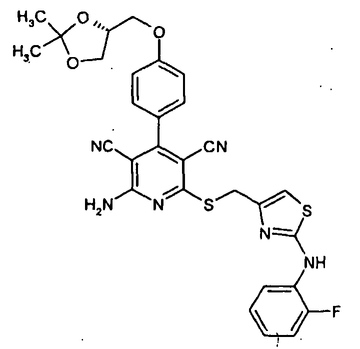 | 589 | 2.80 (4) | 66 |

### Beispiel 29A

### 2-Amino-4-[4-(2-hydroxyethoxy)phenyl]-6-phenylthio-pyridin-3,5-dicarbonitril

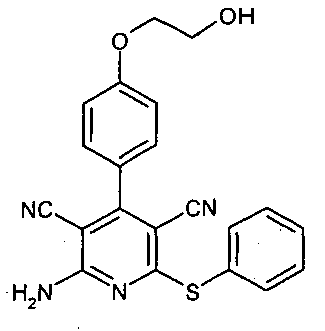

0.765 g (11.6 mmol) Malonsäuredinitril, 1.28 g (11.6 mmol) Thiophenol und 2.48 g (11.6 mmol) 2-[4-(2-Hydroxyethoxy)benzyliden]malononitril [Herstellung gemäß WO 03/053441, Beispiel 6 /Methode 2, 1. Stufe] werden in 15 ml Ethanol gelöst und mit 0.03 ml Triethylamin versetzt. Der Ansatz wird 2 h unter Rückfluss gerührt. Nach dem Abkühlen wird das Reaktionsgemisch filtriert und der Rückstand mit Ethanol gewaschen und getrocknet.
Ausbeute: 2.07 g (46% d. Th.)
¹H-NMR (400 MHz, DMSO-d₆): δ = 7.76 (br. s, 2H), 7.60 (m, 2H), 7.51 (m, 5H), 7.12 (d, 2H), 4.93 (t, 1H), 4.09 (t, 2H), 3.75 (m, 2H).
LC-MS (Methode 3): Rₜ = 2.02 min; MS (ESIpos): m/z = 389 [M+H]⁺.

### Beispiel 30A

### 2-Chlor-4-[4-(2-hydroxyethoxy)phenyl]-6-phenylthio-pyridin-3,5-dicarbonitril

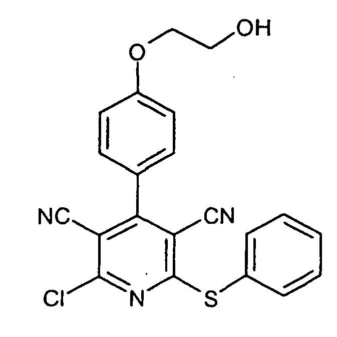

2.07 g (5.33 mmol) der Verbindung aus Beispiel 29A werden in 11 ml absolutem DMF gelost und mit 4.30 g (32.0 mmol) wasserfreiem Kupfer(II)chlorid und 2.71 ml (32.0 mmol) Isoamylnitrit versetzt. Der Ansatz wird 18 h bei 40°C gerührt. Die Reaktionslösung wird dann am Rotationsverdampfer eingeengt und der Rückstand auf 1 N Salzsäure gegeben. Man extrahiert dreimal mit Dichlormethan und wäscht die vereinigten organischen Phasen mit 1 N Salzsäure und Kochsalz-Lösung. Nach dem Trocknen über Magnesiumsulfat wird das Lösungsmittel am Rotationsverdampfer entfernt. Das Rohprodukt wird chromatographisch an Kieselgel 60 (Laufmittel: Dichlormethan/Ethanol 20:1) gereinigt.
Ausbeute: 1.29 g (59% d. Th.)
¹H-NMR (400 MHz, DMSO-d₆): δ = 7.60 (m, 7H), 7.20 (d, 2H), 4.12 (t, 2H), 3.76 (t, 2H).
LC-MS (Methode 3): Rₜ = 2.38 min; MS (ESIpos): m/z = 408 [M+H]⁺.

### Beispiel 31A

### 2-(2-Hydroxyethoxy)amino-4-[4-(2-hydroxyethoxy)phenyl]-6-phenylthio-pyridin-3,5-dicarbonitril

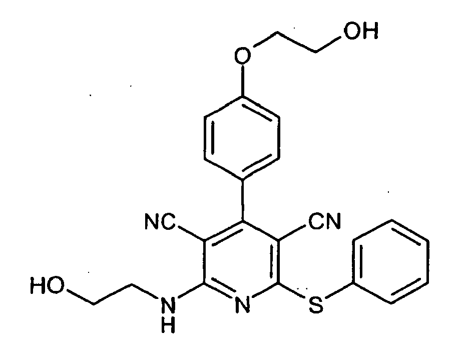

0.50 g (1.23 mmol) der Verbindung aus Beispiel 30A werden in 1.5 ml DMF gelöst und mit 0.16 ml (2.70 mmol) 2-Hydroxyethylamin versetzt. Man lässt für 20 min rühren und gibt dann 2 ml Methanol und 4 ml Wasser hinzu. Der Niederschlag wird abfiltriert, mit Methanol gewaschen und getrocknet.
Ausbeute: 0.36 g (68% d. Th.)
¹H-NMR (400 MHz, DMSO-d₆): δ = 7.94 (br. s, 1H), 7.55 (m, 7H), 7.13 (d, 2H), 4.93 (t, 1H), 4.49 (t, 1H), 4.09 (t, 2H), 3.75 (m, 2H), 3.09 (m, 2H), 3.00 (m, 2H).
LC-MS (Methode 4): Rₜ = 2.33 min; MS (ESIpos): m/z = 433 [M+H]⁺.

### Beispiel 32A

### 2-(2-Hydroxyethoxy)amino-4-[4-(2-hydroxyethoxy)phenyl]-6-mercaptopyridin-3,5-dicarbonitril

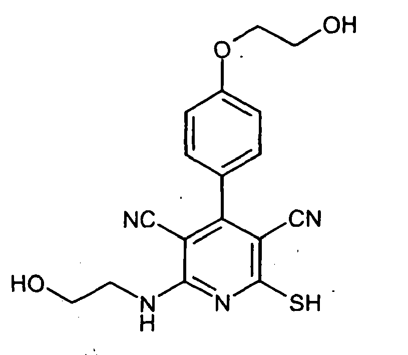

0.30 g (0.70 mmol) der Verbindung aus Beispiel 31A werden in 2 ml DMF gelöst und mit 0.19 g (2.43 mmol) Natriumsulfid versetzt. Der Ansatz wird 2 h bei 80°C und anschließend 12 h bei RT gerührt. Danach gibt man 1 N Salzsäure (10 ml) hinzu und filtriert den Niederschlag ab.
Ausbeute: 0.25 g (72% d. Th.)
LC-MS (Methode 3): Rₜ = 1.21 min; MS (ESIpos): m/z = 357 [M+H]⁺.

### Beispiel 33A

### 2-Amino-6-(benzylthio)-4-{4-[2-(dimethylamino)ethoxy]phenyl}pyridin-3,5-dicarbonitril

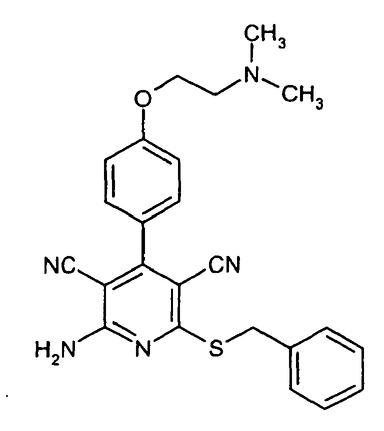

Zu einer Lösung von 8.39 g (23.4 mmol) 2-Amino-6-(benzylthio)-4-(4-hydroxyphenyl)pyridin-3,5-dicarbonitril [Herstellung gemäß WO 01/25210, Beispiel A 383, aus 2-Amino-4-(4-hydroxyphenyl)-6-mercaptopyridin-3,5-dicarbonitril und Benzylbromid] in 105.5 ml Ethanol werden 6.31 g (56.2 mmol) Kalium-*tert*.-butylat gegeben. Es wird 1 h bei RT nachgerührt und dann 4.05 g (28.1 mmol) 2-Dimethylaminoethylchlorid-Hydrochlorid zugegeben. Anschließend rührt man 3 h bei +78°C. Das Reaktionsgemisch wird nach Abkühlen filtriert und das Filtrat am Rotationsverdampfer eingeengt. Die Reinigung des Rückstands erfolgt direkt mittels präparativer HPLC (Säule: Merck 210 g RP Kieselgel Gromsil 120 ODS-4 HR 10 µm, 50 mm x 200 mm; Eluent A = Wasser + 0.1% Ameisensäure, Eluent B = Acetonitril; Gradient: 0 min 10% B → 5 min 10% B → 6 min 90% B → 22 min 90% B → 22 min 10% B → 28 min 10% B; Flussrate: 110 ml/min; Wellenlänge: 220 nm).
Ausbeute: 3.55 g (35% d. Th.)
LC-MS (Methode 3): Rₜ = 1.57 min; MS (ESIpos): m/z = 430 [M+H]⁺.

### Beispiel 34A

### 2-Amino-4-{4-[2-(dimethylamino)ethoxy]phenyl}-6-mercaptopyridin-3,5-dicarbonitril

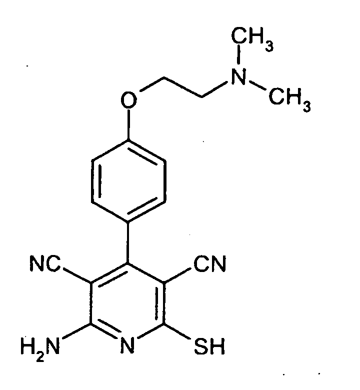

Zu einer Lösung von 3.56 g (8.28 mmol) der Verbindung aus Beispiel 33A in 13 ml trockenem DMF werden 0.97 g (12.41 mmol) Natriumsulfid gegeben. Das Reaktionsgemisch wird 2 h bei +80°C gerührt. Nach Abkühlen auf RT wird das Reaktionsgemisch mit 2 ml 37%-iger Salzsäure versetzt, wobei Erwärmung auf 65°C auftritt. Nach Zugabe von 2.6 ml Wasser wird das Reaktionsgemisch wieder auf RT abgekühlt. Nach Zugabe von weiteren 5 ml Wasser wird mit 5 ml Essigsäureethylester gewaschen und durch Zugabe von 40%-iger Natronlauge alkalisch gestellt. Es fallen gelbe Kristalle aus, die abgesaugt und mit 10 ml Wasser sowie 10 ml Diethylether gewaschen und anschließend im Vakuum getrocknet werden. Das Filtrat wird am Rotationsverdampfer eingeengt und mit wenig Wasser verrührt. Die erhaltenen Kristalle werden abgesaugt, mit je 10 ml Wasser und Diethylether gewaschen und im Vakuum getrocknet.
Ausbeute: 0.38 g (13% d. Th.)
¹H-NMR (400 MHz, DMSO-d₆): δ = 10.77 (br. s, 1H), 7.39 (d, 2H), 7.09 (d, 2H), 6.92 (br. s, 2H), 4.30 (t, 2H), 3.21 (br. s, 2H), 2.64 (s, 6H).
LC-MS (Methode 3): Rₜ = 0.83 min; MS (ESIpos): m/z = 340 [M+H]⁺.

### Beispiel 35A

### 2-(4-Formylphenoxy)ethyl 4-methylphenylsulfonat

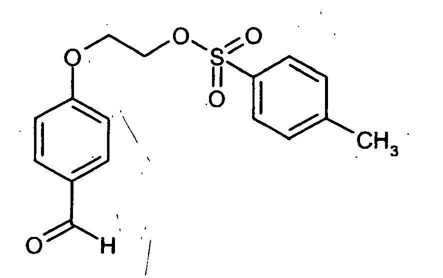

Zu einer Lösung von 10.0 g (60.2 mmol) 4-(2-Hydroxyethoxy)benzaldehyd in 300 ml Dichlormethan werden bei RT nacheinander unter Rühren 12.6 ml (90.3 mmol) Triethylamin und eine Lösung von 13.77 g (72.2 mmol) Toluol-4-sulfonylchlorid in 200 ml Dichlormethan zugetropft. Das Reaktionsgemisch wird 12 h bei RT gerührt. Nach Zugabe von 0.15 g (1.2 mmol) 4-*N,N-*Dimethylaminopyridin wird noch für weitere 4 h bei RT gerührt. Dann werden 250 ml gesättigte wässrige Natriumhydrogencarbonat-Lösung zugegeben und die Mischung dreimal mit je 100 ml Dichlormethan extrahiert. Die vereinigten organischen Phasen werden über Natriumsulfat getrocknet. Nach Entfernen des Lösungsmittels am Rotationsverdampfer wird das Rohprodukt an Kieselgel 60 (Laufmittelgradient Cylohexan → Essigsäureethylester) chromatographisch gereinigt.
Ausbeute: 12.34 g (64% d. Th.)
HPLC (Methode 6): Rₜ = 4.57 min; MS (ESIpos): m/z = 321 [M+H]⁺.

### Beispiel 36A

### 4-(2-Azidoethoxy)benzaldehyd

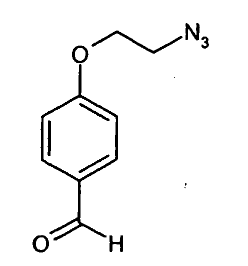

Eine Lösung von 5.0 g (15.61 mmol) der Verbindung aus Beispiel 35A und 2.03 g (31.22 mmol) Natriumazid in 100 ml trockenem DMF wird 12 h bei RT gerührt. Das Reaktionsgemisch wird am Rotationsverdampfer eingeengt und der Rückstand mit ca. 20 ml Wasser suspendiert. Nach dreimaliger Extraktion mit je 30 ml Diethylether werden die vereinigten organischen Phasen zweimal mit jeweils 10 ml Wasser und einmal mit 10 ml gesättigter Kochsalz-Lösung gewaschen. Nach Trocknen über Natriumsulfat wird das Lösungsmittel am Rotationsverdampfer entfernt.
Ausbeute: 3.02 g (98% d. Th.)
HPLC (Methode 7): Rₜ = 4.14 min; MS (DCI): m/z = 209 [M+NH₄]⁺.

### Beispiel 37A

### [4-(2-Azidoethoxy)benzyliden]malononitril

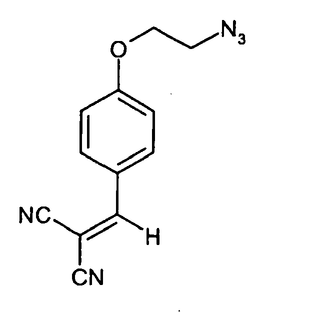

Zu einer Lösung von 3.02 g (15.79 mmol) der Verbindung aus Beispiel 36A und 1.09 g (16.42 mmol) Malonsäuredinitril in 100 ml Ethanol werden 47 µl (0.47 mmol) Piperidin getropft und das Reaktionsgemisch 3.5 h lang bei +78°C gerührt. Die Lösung färbt sich hierbei orange-rot. Nach Abkühlen auf RT wird die Lösung ohne Rühren 20 h lang stehengelassen. Es bildet sich ein farbloser Niederschlag. Die Rohsuspension wird am Rotationsverdampfer auf die Hälfte des ursprunglichen Volumens eingeengt und die Kristallisation unter Kühlung im Eisbad vervollständigt. Der erhaltene Niederschlag wird abgesaugt und zweimal mit je 20 ml Ethanol und zweimal mit je 20 ml Methyl-*tert*.-butylether nachgewaschen.
Ausbeute: 2.38 g (63% d. Th.)
MS (DCI): m/z = 257 [M+NH₄]⁺.

### Beispiel 38A

### 2-Amino-4-[4-(2-azidoethoxy)phenyl]-6-mercaptopyridin-3,5-dicarbonitril

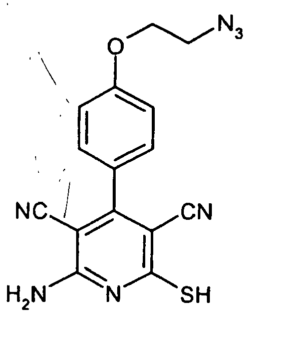

Eine Lösung von 2.39 g (9.98 mmol) der Verbindung aus Beispiel 37A und 2.50 g (24.92 mmol) Cyanothioacetamid in 100 ml Ethanol wird 6 h bei +78°C gerührt. Nach Abkühlen auf RT und 12 h weiterem Stehenlassen ohne Rühren wird das Reaktionsgemisch am Rotationsverdampfer eingeengt. Der Rückstand wird aus ca. 30 ml Ethanol umkristallisiert. Der erhaltene Niederschlag wird abgesaugt und zweimal mit je 10 ml Methyl-*tert*.-butylether nachgewaschen.
Ausbeute: 2.04 g (61 % d. Th.)
MS (DCI): m/z = 355 [M+NH₄]⁺.

### Beispiel 39A

### 2-Amino-4-[4-(2-azidoethoxy)phenyl]-6-[({2-[(4-fluorphenyl)amino]-1,3-thiazol-4-yl}methyl)-thio]pyridin-3,5-dicarbonitril

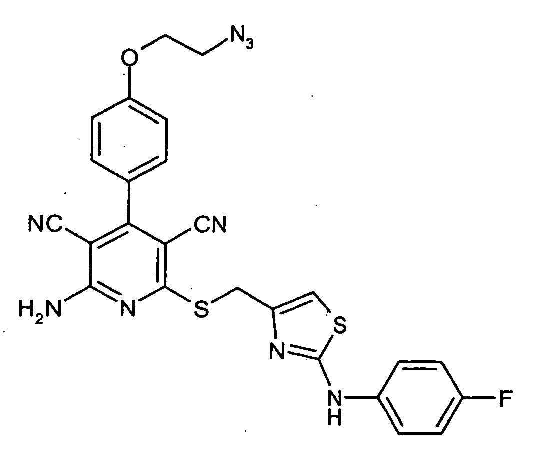

Eine Lösung von 74 mg (0.44 mmol) 4-Fluorphenylthioharnstoff und 55 mg (0.44 mmol) 1,3-Dichloraceton in 5 ml Ethanol wird 60 min bei +85°C gerührt. Nach Entfernen des Lösungsmittels am Rotationsverdampfer wird der Rückstand in 5 ml DMF aufgenommen, mit 105 mg (0.31 mmol) der Verbindung aus Beispiel 38A und 78 mg (0.93 mmol) Natriumhydrogencarbonat versetzt und dann 20 h bei RT gerührt. Anschließend wird der Ansatz auf 15 ml gesättigte Natriumhydrogencarbonat-Lösung gegeben. Man extrahiert mit Essigsäureethylester (dreimal je 15 ml) und trocknet die vereinigten organischen Phasen über Magnesiumsulfat. Nach Entfernen des Lösungsmittels am Rotationsverdampfer wird das Rohprodukt an Kieselgel 60 (Laufmittelgradient Dichlormethan/Ethanol 200:1 → 20: 1) chromatographisch gereinigt.
Ausbeute: 79 mg (47% d. Th.)
¹H-NMR (400 MHz, DMSO-d₆): δ = 10.22 (s, 1H), 8.27-7.86 (br. s, 2H), 7.66-7.58 (m, 2H), 7.50 (d, 2H), 7.17-7.08 (m, 4H), 6.96 (s, 1H), 4.46 (s, 2H), 4.31-4.22 (m, 2H), 3.74-3.67 (m, 2H).
LC-MS (Methode 2): Rₜ = 2.72 min; MS (ESIpos): m/z = 544 [M+H]⁺.

### Beispiel 40A

### 4-(2-Hydroxypropoxy)benzaldehyd

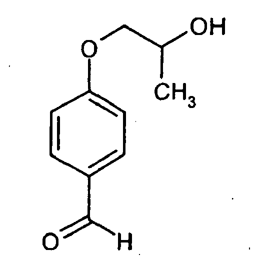

Zu einer Lösung von 7.03 g (57.5 mmol) *p*-Hydroxybenzaldehyd und 6.80 g (71.9 mmol) 1-Chlor-2-propanol (techn., ca. 70%, Isomerengemisch mit 2-Chlor-1-propanol) in 125 ml trockenem DMF werden 18.30 g (172.6 mmol) Natriumcarbonat gegeben und der Ansatz 20 h bei +130°C gerührt. Nach Abkühlen auf RT wird das Gemisch mit 100 ml gesättigter Natriumhydrogencarbonat-Lösung versetzt und mit Essigsäureethylester (dreimal je 100 ml) extrahiert. Die vereinigten organischen Phasen werden über Magnesiumsulfat getrocknet. Nach Entfernen des Lösungsmittels am Rotationsverdampfer wird das Rohprodukt an Kieselgel 60 (Laufmittelgradient Cyclohexan/Essigsäureethylester 5:1 → 2:1) chromatographisch gereinigt.
Ausbeute: 4.60 g (44% d. Th., Isomerengemisch im Verhältnis 75:25)
LC-MS (Methode 4): Rₜ = 1.63 min; MS (ESIpos): m/z = 181 [M+H]⁺.

### Beispiel 41A

### 4-(2-{[tert.-Butyl(dimethyl)silyl]oxy}propoxy)benzaldehyd

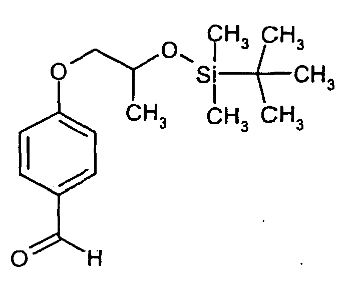

Zu einer Lösung von 4.60 g (25.5 mmol) der Verbindung aus Beispiel 40A in 120 ml trockenem Dimethylformamid werden nacheinander 5.39 g (35.7 mmol) *tert*.-Butyldimethylsilylchlorid und 3.30 g (48.5 mmol) Imidazol gegeben und das Gemisch 20 h lang bei RT gerührt. Anschließend wird die Reaktionsmischung mit 100 ml gesättigter Natriumhydrogencarbonat-Lösung versetzt und mit Diethylether (dreimal je 100 ml) extrahiert. Die vereinigten organischen Phasen werden über Magnesiumsulfat getrocknet. Nach Entfernen des Lösungsmittels am Rotationsverdampfer wird das Rohprodukt an Kieselgel 60 (Laufmittelgradient Cyclohexan/Essigsäureethylester 50:1 → 10:1) chromatographisch gereinigt.
Ausbeute: 4.00 g (53% d. Th., Isomerengemisch im Verhältnis 86:14)
LC-MS (Methode 2): Rₜ = 3.29 min; MS (ESIpos): m/z = 295 [M+H]⁺.

### Beispiel 42A

### 2-Amino-4-[4-(2-{[tert.-butyl(dimethyl)silyl]oxy}propoxy)phenyl]-6-mercaptopyridin-3,5-dicarbonitril

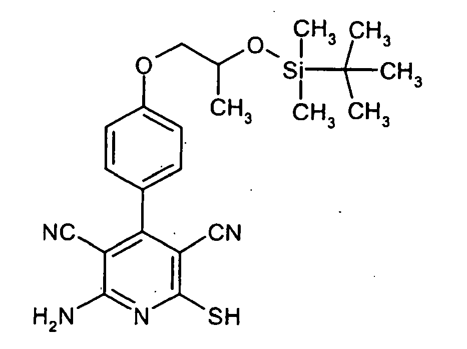

Eine Lösung von 1.77 g (5.99 mmol) der Verbindung aus Beispiel 41A und 1.26 g (12.59 mmol) Cyanothioacetamid in 25 ml Ethanol wird 6 h lang bei +78°C gerührt. Anschließend wird auf RT abgekühlt und für weitere 20 h bei dieser Temperatur gerührt. Der ausgefallene Niederschlag wird abgesaugt und mit kaltem Diethylether nachgewaschen. Weiteres Produkt wird aus der eingeengten Filtratlösung durch chromatographische Reinigung an Kieselgel 60 (Laufmittelgradient Cyclohexan/Essigsäureethylester 1:1 → 1:4) erhalten.
Ausbeute: 0.25 g (9% d. Th., Isomerengemisch)
LC-MS (Methode 3): Rₜ = 2.71 min, 2.77 min; MS (ESIpos): m/z = 430 [M+H]⁺.

### Beispiel 43A

### 2-Amino-4-[4-(2-{[tert.-butyl(dimethyl)silyl]oxy}propoxy)phenyl]-6-[({2-[(4-fluorphenyl)amino]-1,3-thiazol-4-yl}methyl)thio]pyridin-3,5-dicarbonitril

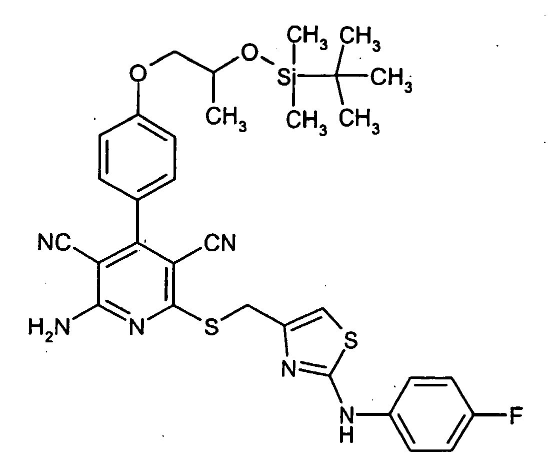

Eine Lösung von 78.6 mg (0.46 mol) 4-Fluorphenylthioharnstoff und 56.0 mg (0.44 mmol) 1,3-Dichloraceton in 2 ml trockenem DMF wird 3 h bei +80°C gerührt. Nach Abkühlen auf RT werden 370 mg (0.42 mmol) der Verbindung aus Beispiel 42A zugegeben und der Ansatz anschließend für 20 h bei RT gerührt. Das Reaktionsgemisch wird direkt über zweimalige präparative HPLC (Säule: YMC GEL ODS-AQ S-5 / 15 µm; Laufmittelgradient: Acetonitril/Wasser 10:90 → 95:5) gereinigt.
Ausbeute: 0.44 g (14% d. Th.)
¹H-NMR (400 MHz, DMSO-d₆): δ = 10.21 (s, 1H), 8.18-7.93 (br. s, 2H), 7.60 (dd, 2H), 7.47 (d, 2H), 7.12 (t, 2H), 7.07 (d, 2H), 6.95 (s, 1H), 4.44 (s, 2H), 4.21-4.12 (m, 1H), 3.96 (dd, 1H), 3.87 (dd, 1H), 1.18 (d, 3H), 0.87 (s, 9H), 0.08 (d, 6H).
LC-MS (Methode 5): Rₜ = 7.35 min; MS (ESIpos): m/z = 647 [M+H]⁺.

### Ausführungsbeispiele:

### Beispiel 1

### 2-Amino-6-[({2-[(3-chlorphenyl)amino]-1,3-thiazol-4-yl}methyl)thio]-4-[4-(2,3-dihydroxypropoxy)phenyl]pyridin-3,5-dicarbonitril

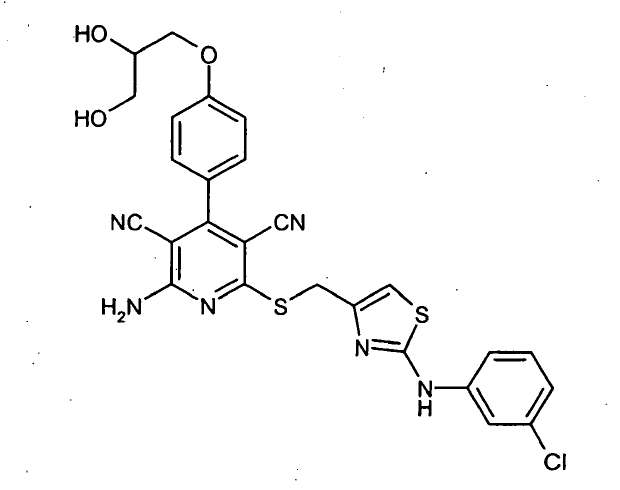

Zu 1 ml Wasser werden 2 ml Eisessig und 90 mg (0.15 mmol) der Verbindung aus Beispiel 7A gegeben und der Ansatz für 16 h bei RT gerührt. Man engt ein und chromatographiert den Rückstand an Kieselgel 60 (Laufmittelgradient Dichlormethan → Dichlonnethan/Methanol 10:1).
Ausbeute: 30 mg (36% d. Th.)
Fp.: 192-194°C
¹H-NMR (400 MHz, DMSO-d₆): δ = 10.42 (s, 1H), 8.06 (br. s, 2H), 7.82 (s, 1H), 7.45 (m, 3H), 7.30 (t, 1H), 7.10 (d, 2H), 7.03 (s, 1H), 6.97 (d, 1H), 4.99 (d, 1H), 4.68 (dd, 1H), 4.49 (s, 2H), 4.09 (dd, 1H), 3.95 (dd, 1H), 3.82 (m, 1H), 3.46 (dd, 2H).
LC-MS (Methode 3): Rₜ = 2.17 min; MS (ESIpos): m/z = 565 [M+H]⁺.

Die in Tabelle 3 aufgeführten Beispiele werden aus den entsprechenden Ausgangsverbindungen analog zu Beispiel 1 hergestellt:

**Tabelle 3**

| **Beispiel Nr.** | **Struktur** | **MS (ESI) [M+H]⁺** | **LC-MS Rₜ [min] (Methode)** | **¹H-NMR** | **Ausbeute (% d. Th.)** |
|---|---|---|---|---|---|
| **2** | 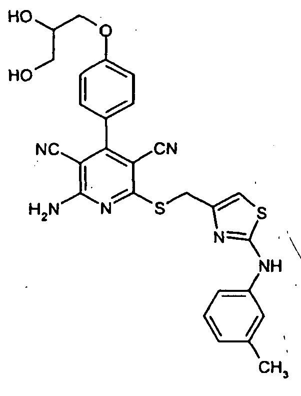 | 545 | 2.29 (2) | | 77 |
| **3** | 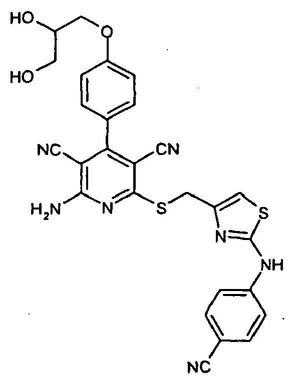 | 556 | 2.16 (2) | | 63 |
| **4** | 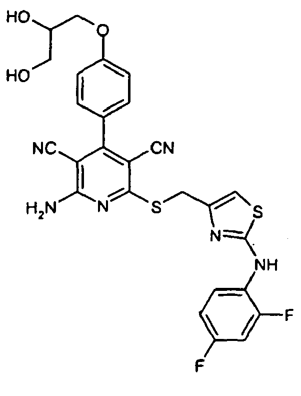 | 567 | 2.26 (2) | | 98 |
| **5** | 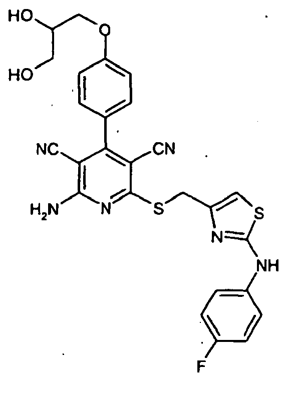 | 549 | 2.30 (4) | δ (400 MHz, DMSO-d₆) = 10.24 (s, 1H), 8.09 (br. s, 2H), 7.61 (dd, 2H), 7.46 (d, 2H), 7.18-7.05 (m, 4H), 6.97 (s, 1H), 5.00 (d, 1H), 4.70 (dd, 1H), 4.45 (s, 2H), 4.09 (dd, 1H), 3.95 (dd, 1H), 3.81 (m, 1H), 3.46 (dd, 2H). | 83 |
| **6** | 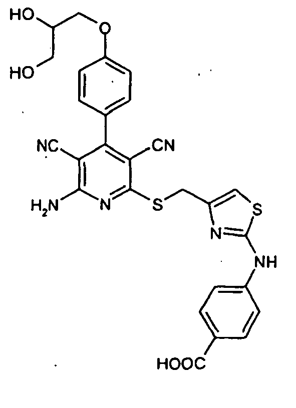 | 575 | 2.02 (4) | δ (400 MHz, DMSO-d₆) = 10.63 (s, 1H), 8.10 (br. s, 2H), 7.86 (d, 2H), 7.67 (d, 2H), 7.49 (d, 2H), 7.10 (d, 2H), 7.08 (s, 1H), 5.01 (br. s, 1H), 4.71 (br. m, 1H), 4.09 (dd, 1H), 3.95 (dd, 1H), 3.81 (m, 1H), 3.46 (d, 1H), 3.04 (m, 1H). | 67 |
| **7** | 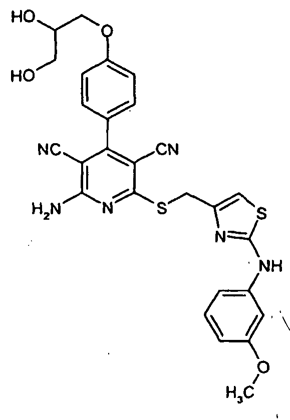 | 561 | 2.28 (4) | | 87 |

### Beispiel 8

### 2-Amino-6-[({2-[(4-fluorphenyl)amino]-1,3-thiazol-4-yl}methyl)thio]-4-[4-(2-hydroxyethoxy)-phenyl]pyridin-3,5-dicarbonitril

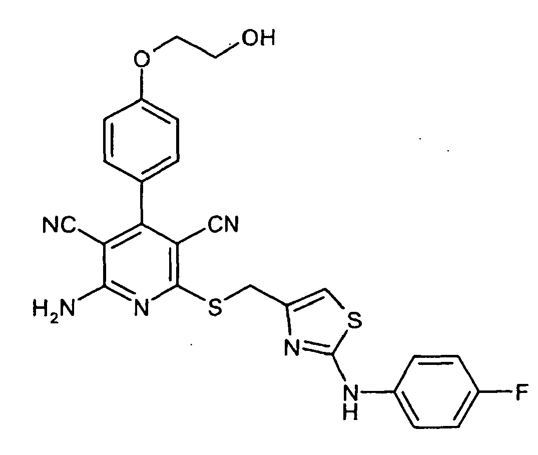

Zu einer Lösung von 327 mg (1.92 mmol) 4-Fluorphenylthioharnstoff in 8 ml Ethanol werden 244 mg (1.92 mmol) 1,3-Dichloraceton gegeben und der Ansatz 1 h unter Rückfluss gerührt. Man engt ein, löst den Rückstand in 4 ml DMF, gibt 429 mg (1.37 mmol) 2-Amino-4-[4-(2-hydroxyethoxy)-phenyl]-6-mercaptopyridin-3,5-dicarbonitril (Herstellung siehe WO 03/053441, Beispiel 6 /Methode 1, 1. Stufe) und 346 mg (4.1 mmol) Natriumhydrogencarbonat hinzu und rührt über Nacht bei RT. Nach Zugabe von Wasser wird vom Niederschlag abdekantiert und der Rückstand mit Dichlormethan verrieben. Nach Chromatographie an Kieselgel (Laufmittel Dichlormethan/Methanol 50:1) erhält man die Titelverbindung als gelblichen Feststoff.
Ausbeute: 316 mg (44% d. Th.)
HPLC (Methode 1): Rₜ = 4.24 min
¹H-NMR (400 MHz, DMSO-d₆): δ = 10.23 (s, 1H), 8.1 (br. s, 2H), 7.62 (dd, 2H), 7.47 (d, 2H), 7.12 (dd, 4H), 6.96 (s, 1H), 4.92 (t, 1H), 4.45 (s, 2H), 4.07 (t, 2H), 3.74 (q, 2H).
LC-MS (Methode 2): Rₜ = 2.39 min; MS (ESIpos): m/z = 519 [M+H]⁺.

### Beispiel 9

### 2-Amino-6-[({2-[(4-chlorphenyl)amino]-1,3-thiazol-4-yl}methyl)thio]-4-[4-(2-hydroxyethoxy)-phenyl]pyridin-3,5-dicarbonitril

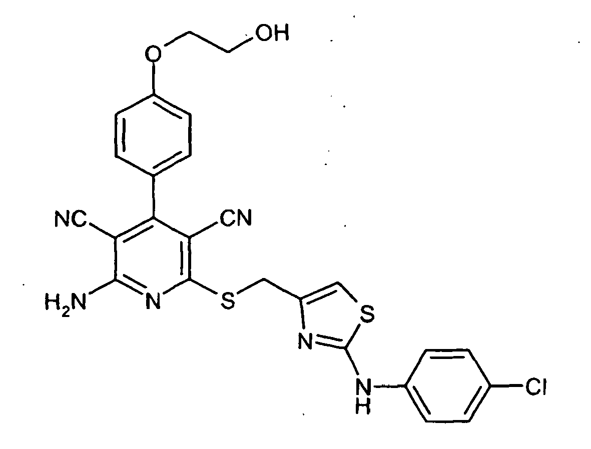

Analog zu Beispiel 8 erhält man die Titelverbindung durch Reaktion von 179 mg (0.96 mmol) 4-Chlorphenylthiohamstoff mit 122 mg (0.96 mmol) 1,3-Dichloraceton in Ethanol und anschliessende Umsetzung mit 150 mg (0.48 mmol) 2-Amino-4-[4-(2-hydroxyethoxy)phenyl]-6-mercaptopyridin-3,5-dicarbonitril.
Ausbeute: 60 mg (12% d. Th.)
HPLC (Methode 1): Rₜ = 4.44 min
¹H-NMR (400 MHz, DMSO-d₆): δ = 10.37 (s, 1H), 8.1 (br. s, 2H), 7.63 (d, 2H), 7.47 (d, 2H), 7.32 (d, 2H), 7.11 (d, 2H), 6.99 (s, 1H), 4.47 (s, 2H), 4.08 (t, 2H), 3.75 (q, 2H).
LC-MS (Methode 3): Rₜ = 2.31 min; MS (ESIpos): m/z = 535 [M+H]⁺.

### Beispiel 10

### 2-Amino-6-[({2-[(2,4-difluorphenyl)amino]-1,3-thiazol-4-yl}methyl)thio]-4-[4-(2-hydroxyethoxy)-phenyl]pyridin-3,5-dicarbonitril

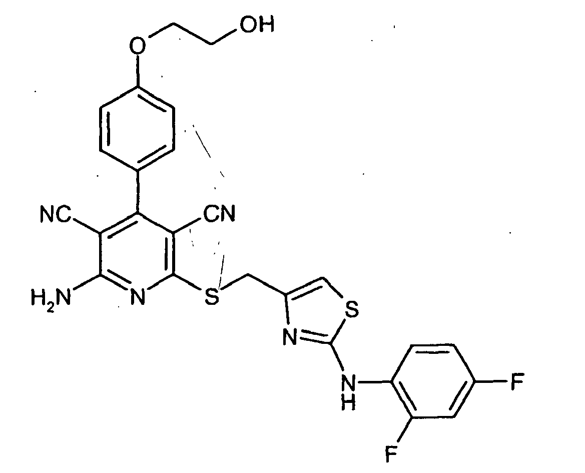

Analog zu Beispiel 8 erhält man die Titelverbindung durch Reaktion von 169 mg (0.90 mmol) 2,4-Difluorphenylthiohamstoff mit 114 mg (0.90 mmol) 1,3-Dichloraceton in Ethanol und anschließende Umsetzung mit 200 mg (0.64 mmol) 2-Amino-4-[4-(2-hydroxyethoxy)phenyl]-6-mercaptopyridin-3,5-dicarbonitril.
Ausbeute: 126 mg (36% d. Th.)
HPLC (Methode 1): Rₜ = 4.31 min
¹H-NMR (400 MHz, DMSO-d₆): δ = 9.97 (s, 1H), 8.34 (dt, 1H), 8.1 (br. s, 2H), 7.47 (d, 2H), 7.30 (dq, 1H), 7.10 (d, 2H), 7.04 (br. t, 1H), 6.99 (s, 1H), 4.91 (t, 1H), 4.45 (s, 2H), 4.06 (t, 2H), 3.74 (q, 2H).
LC-MS (Methode 3): Rₜ = 2.21 min; MS (ESIpos): m/z = 537 [M+H]⁺.

### Beispiel 11

### 2-Amino-6-[({2-[(3-fluorphenyl)amino]-1,3-thiazol-4-yl}methyl)thio]-4-[4-(2-hydroxyethoxy) phenyl]pyridin-3,5-dicarbonitril

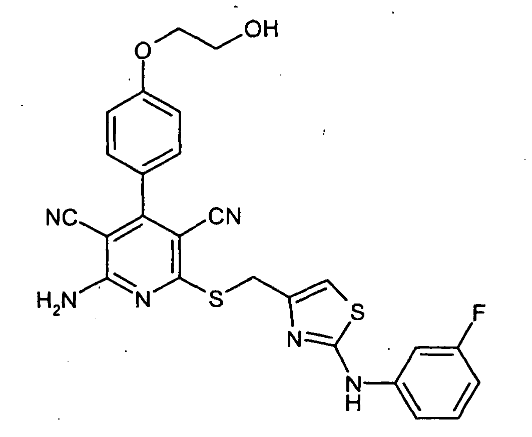

Analog zu Beispiel 8 erhält man die Titelverbindung durch Reaktion von 153 mg (0.90 mmol) 3-Fluorphenylthioharnstoff mit 114 mg (0.90 mmol) 1,3-Dichloraceton in Ethanol und anschließende Umsetzung mit 200 mg (0.64 mmol) 2-Amino-4-[4-(2-hydroxyethoxy)phenyl]-6-mercaptopyridin-3,5-dicarbonitril.
Ausbeute: 80 mg (24% d. Th.)
HPLC (Methode 1): Rₜ = 4.36 min
¹H-NMR (400 MHz, DMSO-d₆): δ = 10.46 (s, 1H), 8.1 (br. s, 2H), 7.66 (dt, 1H), 7.47 (d, 2H), 7.35-7.21 (m, 2H), 7.10 (t, 2H), 7.04 (s, 1H), 6.74 (dt, 1H), 4.92 (br. s, 1H), 4.48 (s, 2H), 4.07 (t, 2H), 3.74 (t, 2H).
LC-MS (Methode 3): Rₜ = 2.20 min; MS (ESIpos): m/z = 519 [M+H]⁺.

### Beispiel 12

### 2-Amino-6-[({2-[(2-fluorphenyl)amino]-1,3-thiazol-4-yl}methyl)thio]-4-[4-(2-hydroxyethoxy)-phenyl]pyridin-3,5-dicarbonitril

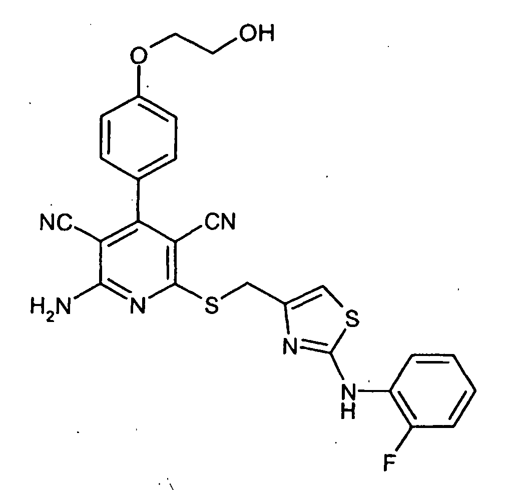

Analog zu Beispiel 8 erhält man die Titelverbindung durch Reaktion von 153 mg (0.90 mmol) 2-Fluorphenylthioharnstoff mit 114 mg (0.90 mmol) 1,3-Dichloraceton in Ethanol und anschließende Umsetzung mit 200 mg (0.64 mmol) 2-Amino-4-[4-(2-hydroxyethoxy)phenyl]-6-mercaptopyridin-3,5-dicarbonitril.
Ausbeute: 170 mg (51% d. Th.)
HPLC (Methode 1): Rₜ = 4.28 min
¹H-NMR (400 MHz, DMSO-d₆): δ = 9.99 (s, 1H), 8.36 (t, 1H), 8.1 (br. s, 2H), 7.46 (d, 2H), 7.22 (dd, 1H), 7.16-7.08 (m, 3H), 7.02-6.96 (m, 2H), 4.90 (t, 1H), 4.46 (s, 2H), 4.07 (t, 2H), 3.74 (t, 2H).
LC-MS (Methode 3): Rₜ = 2.16 min; MS (ESIpos): m/z = 519 [M+H]⁺.

### Beispiel 13

### 4-({4-[({6-Amino-3,5-dicyano-4-[4-(2-hydroxyethoxy)phenyl]pyridin-2-yl}thio)methyl]-1,3-thiazol-2-yl}amino)benzoesäure

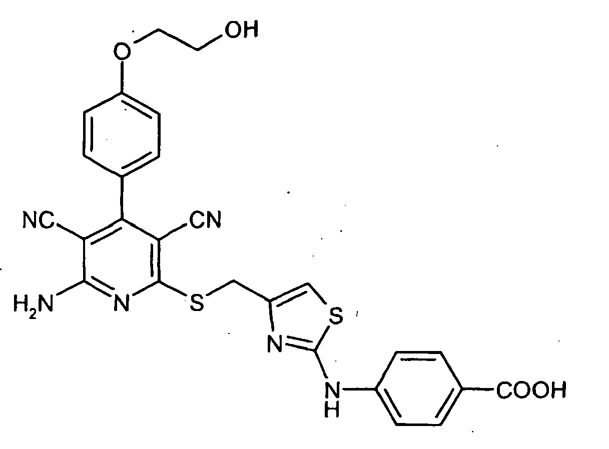

Analog zu Beispiel 8 erhält man die Titelverbindung durch Reaktion von 176 mg (0.90 mmol) 4-[(Aminocarbonothioyl)amino]benzoesäure mit 114 mg (0.90 mmol) 1,3-Dichloraceton in Ethanol und anschließende Umsetzung mit 200 mg (0.64 mmol) 2-Amino-4-[4-(2-hydroxyethoxy)phenyl]-6-mercaptopyridin-3,5-dicarbonitril.
Ausbeute: 45 mg (13% d. Th.)
HPLC (Methode 1): Rₜ = 3.81 min
¹H-NMR (300 MHz, DMSO-d₆): δ = 12.6 (br. s, 1H), 10.64 (s, 1H), 8.1 (br. s, 2H), 7.87 (d, 2H), 7.68 (d, 2H), 7.49 (d, 2H), 7.13-7.06 (m, 3H), 4.45 (s, 2H), 4.07 (t, 2H), 3.74 (t, 2H).
LC-MS (Methode 2): Rₜ = 1.97 min; MS (ESIpos): m/z = 545 [M+H]⁺.

Als Nebenkomponente entsteht bei dieser Umsetzung 4-({4-[({6-Amino-3,5-dicyano-4-[4-(2-hydroxyethoxy)phenyl]pyridin-2-yl}thio)methyl]-1,3-thiazol-2-yl}amino)benzoesäureethylester (siehe Beispiel 14).

### Beispiel 14

### 4-({4-[({6-Amino-3,5-dicyano-4-[4-(2-hydroxyethoxy)phenyl]pyridin-2-yl}thio)methyl]-1,3-thiazol-2-yl}amino)benzoesäureethylester

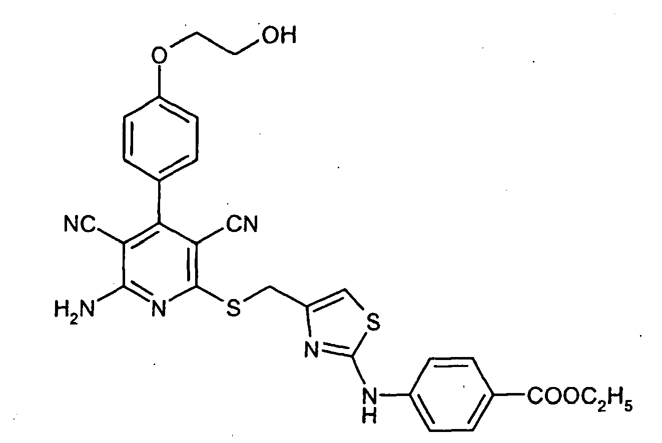

Wie bei Beispiel 13 beschrieben, erhält man die Titelverbindung als Nebenkomponente bei der Reaktion von 176 mg (0.90 mmol) 4-[(Aminocarbonothioyl)amino]benzoesäure mit 114 mg (0.90 mmol) 1,3-Dichloraceton in Ethanol und anschließender Umsetzung mit 200 mg (0.64 mmol) 2-Amino-4-[4-(2-hydroxyethoxy)phenyl]-6-mercaptopyridin-3,5-dicarboniuil.
Ausbeute: 59 mg (16% d. Th.)
HPLC (Methode 1): Rₜ = 4.32 min
¹H-NMR (300 MHz, DMSO-d₆): δ = 10.67 (s, 1H), 8.1 (br. s, 2H), 7.88 (d, 2H), 7.68 (d, 2H), 7.47 (d, 2H), 7.13-7.07 (m, 3H), 4.91 (br. s, 1H), 4.50 (s, 2H), 4.26 (q, 2H), 4.07 (t, 2H), 3.74 (t, 2H), 1.29 (t, 3H).
LC-MS (Methode 2): Rₜ = 2.46 min; MS (ESIpos): m/z = 573 [M+H]⁺.

### Beispiel 15

### 2-Amino-4-[4-(2-hydroxyethoxy)phenyl]-6-[({2-[(4-nitrophenyl)amino]-1,3-thiazol-4-yl}methyl)-thio]pyridin-3,5-dicarbonitril

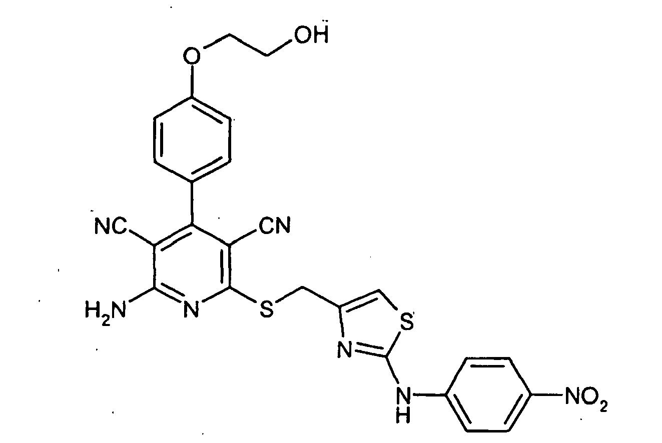

Analog zu Beispiel 8 erhält man die Titelverbindung durch Reaktion von 177 mg (0.90 mmol) 4-Nitrophenylthioharnstoff mit 114 mg (0.90 mmol) 1,3-Dichloraceton in Ethanol und anschließende Umsetzung mit 200 mg (0.64 mmol) 2-Amino-4-[4-(2-hydroxyethoxy)phenyl]-6-mercaptopyridin-3,5-dicarbonitril.
Ausbeute: 67 mg (19% d. Th.)
HPLC (Methode 1): Rₜ = 4.23 min
¹H-NMR (400 MHz, DMSO-d₆): δ = 11.03 (s, 1H), 8.20 (d, 2H), 8.1 (br. s, 2H), 7.80 (d, 2H), 7.48 (d, 2H), 7.20 (s, 1H), 7.10 (d, 2H), 4.90 (t, 1H), 4.52 (s, 2H), 4.07 (t, 2H), 3.74 (q, 2H).
LC-MS (Methode 2): Rₜ = 2.39 min; MS (ESIpos): m/z = 546 [M+H]⁺.

### Beispiel 16

### 2-Amino-4-[4-(2-hydroxyethoxy)phenyl]-6-{[(2-{[3-(trifluormethyl)phenyl]amino}-1,3-thiazol-4-yl)methyl]thio}pyridin-3,5-dicarbonitril

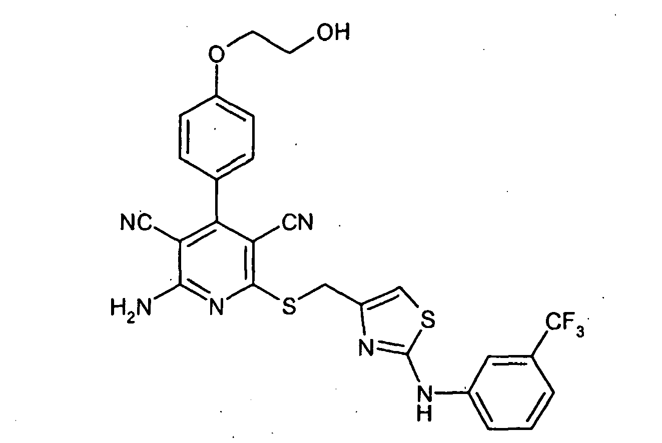

44 mg (0.2 mmol) 3-Trifluormethylthioliarnstoff werden mit einer Lösung von 25.4 mg (0.2 mmol) 1,3-Dichloraceton in 0.5 ml DMF versetzt. Die Reaktionsmischung wird 3 h bei 80°C gerührt. Nach Abkühlen werden 62.5 mg (0.2 mmol) 2-Amino-4-[4-(2-hydroxyethoxy)phenyl]-6-mercaptopyridin-3,5-dicarbonitril in 0.2 ml DMF und 67 mg (0.8 mmol) Natriumhydrogencarbonat zugegeben. Nach Rühren bei RT über Nacht wird die Reaktionsmischung filtriert und durch präparative HPLC gereinigt (Säule: GROMSIL 120 ODS-HE-4, 5 µm, 20 x 50 mm; UV-Detektion: 220 nm; 700 µl Injektionsvolumen; Eluent A: Wasser + 0.1% Ameisensäure, Eluent B: Acetonitril; Gradient: 0 min 10% B → 1.5 min 10% B → 5.5 min 90% B → 7 min 90% B → 7.1 min 10% B → 8 min 10% B; Flussrate: 25 ml/min). Die produkthaltigen Fraktionen werden im Vakuum eingeengt.
Ausbeute: 71.6 mg (63% d. Th.)
LC-MS (Methode 2): Rₜ = 2.56 min; MS (ESIpos): m/z = 569 [M+H]⁺.
¹H-NMR (400 MHz, DMSO-d₆): δ = 10.6 (s, 1H), 8.1 (s, 1H), 8.1 (br. s, 2H), 7.8 (d, 1H), 7.5 (m, 3H), 7.25 (d, 1H), 7.1 (m, 3H), 4.9 (t, 1H), 4.5 (s, 2H), 4.1 (t, 2H), 3.75 (q, 2H).

Analog zu Beispiel 16 werden die in Tabelle 4 aufgeführten Beispiele 17 bis 28 aus 2-Amino-4-[4-(2-hydroxyethoxy)phenyl]-6-mercaptopyridin-3,5-dicarbonitril (Beispiele 17 bis 25) bzw. aus 2-Amino-4-[4-(2-methoxyethoxy)phenyl]-6-mercaptopyridin-3,5-dicarbonitril (Herstellung siehe WO 03/053441, Beispiel 1 / 2. Stufe) (Beispiele 26 bis 28) hergestellt:

**Tabelle 4**

| **Beispiel Nr.** | **Struktur** | **MS (ESI) [M+H]⁺** | **LC-MS Rₜ (min) (Methode)** | **Ausbeute (% d. Th.)** |
|---|---|---|---|---|
| **17** | 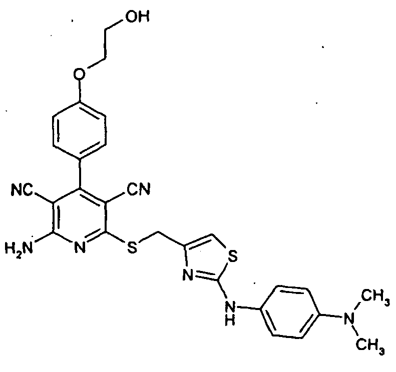 | 544 | 1.8 (2) | 29 |
| **18** | 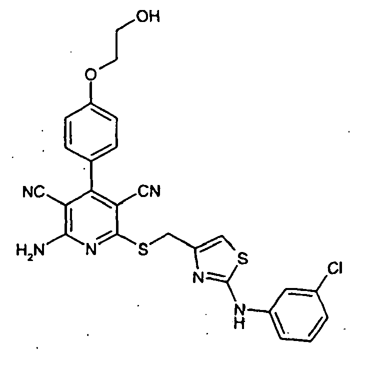 | 536 | 2.5 (2) | 65 |
| **19** | 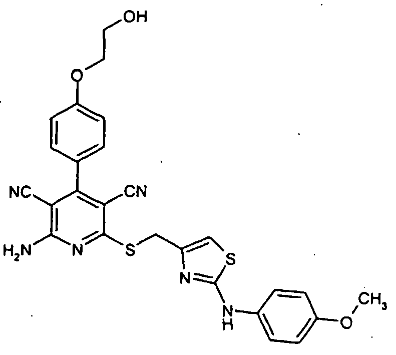 | 531 | 2.28 (2) | 65 |
| **20** | 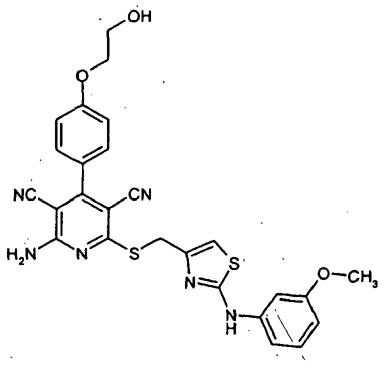 | 531 | 2.35 (2) | 76 |
| **21** | 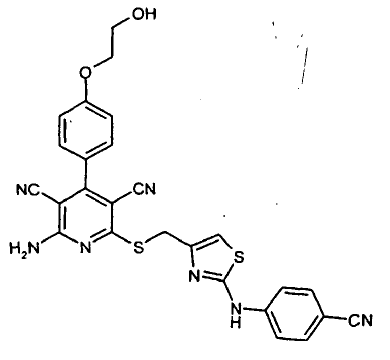 | 526 | 2.31 (2) | 32 |
| **22** | 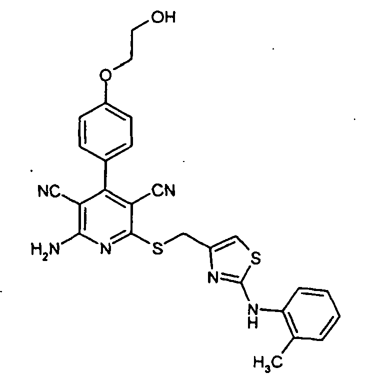 | 515 | 2.36 (2) | 77 |
| **23** | 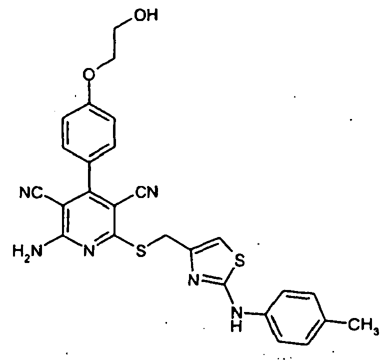 | 515 | 2.44 (2) | 64 |
| **24** | 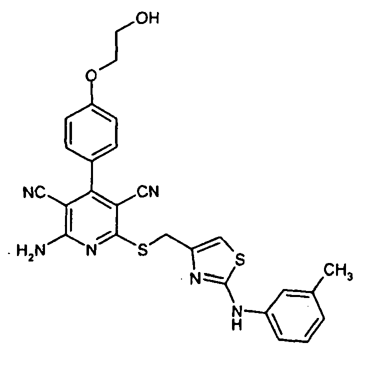 | 515 | 2.44 (2) | 78 |
| **25** | 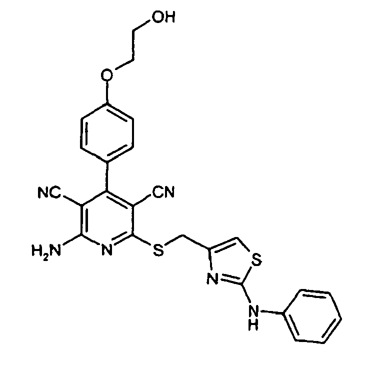 | 501 | 2.35 (2) | 70 |
| **26** | 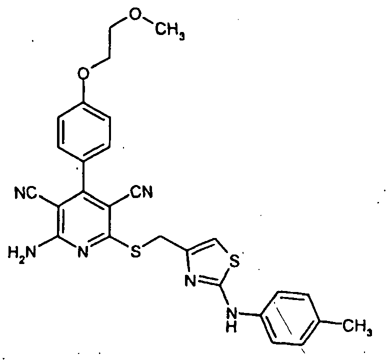 | 529 | 2.74 (4) | 27 |
| **27** | 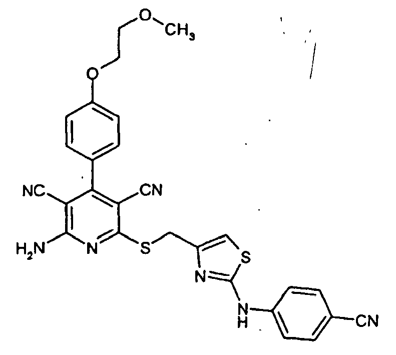 | 540 | 2.60 (4) | 51 |
| **28** | 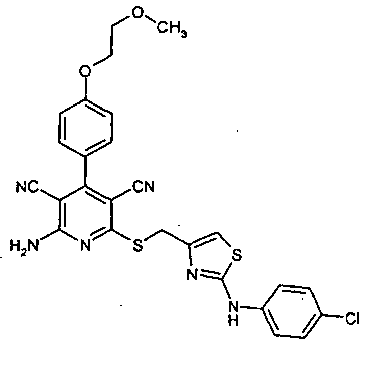 | 550 | 2.79 (2) | 12 |

Die in Tabelle 5 aufgeführten Beispiele werden aus den entsprechenden Ausgangsverbindungen analog zu Beispiel 1 hergestellt:

**Tabelle 5**

| **Beispiel Nr.** | **Struktur** | **MS (ESI) [M+H]⁺** | **LC-MS Rₜ [min] (Methode)** | **¹H-NMR** | **Ausbeute (% d. Th.)** |
|---|---|---|---|---|---|
| **29** | 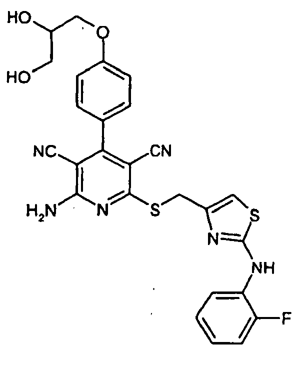 | 549 | 2.25 (4) | δ (400 MHz, DMSO-d₆) = 10.00 (s, 1H), 8.37 (t, 1H), 8.07 (br. s, 2H), 7.47 (d, 2H), 7.23 (m, 1H), 7.12 (m, 3H), 6.99 (m, 2H), 4.47 (s, 2H), 4.09 (dd, 1H), 3.95 (dd, 1H), 3.82 (m, 1H), 3.46 (dd, 2H). | 68 |
| **30** | 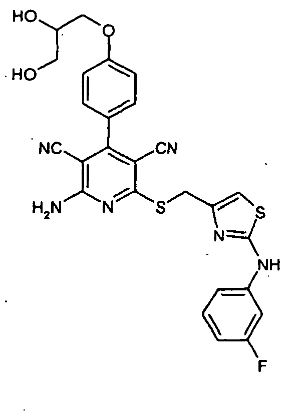 | 549 | 2.32 (4) | δ (400 MHz, DMSO-d₆) = 10.42 (s, 1H), 8.06 (br. s, 2H), 7.82 (s, 1H), 7.45 (m, 3H), 7.30 (t, 1H), 7.10 (d, 2H), 7.03 (s, 1H), 6.74 (dt, 1H), 4.49 (s, 2H), 4.08 (dd, 1H), 3.95 (dd, 1H), 3.82 (m, 1H), 3.46 (dd, 2H). | 60 |
| **31** | 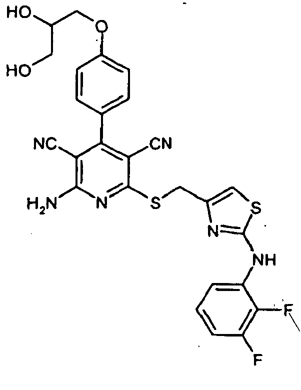 | 567 | 2.32 (4) | δ (400 MHz, DMSO-d₆) = 10.23 (s, 1H), 8.22 (t, 1H), 8.07 (br. s, 2H), 7.46 (d, 2H), 7.09 (m, 4H), 6.99 (dd, 1H), 4.99 (d, 1H), 4.67 (t, 1H), 4.47 (s, 2H), 4.08 (dd, 1H), 3.95 (dd, 1H), 3.81 (m, 1H), 3.46 (t, 2H). | 75 |
| **32** | 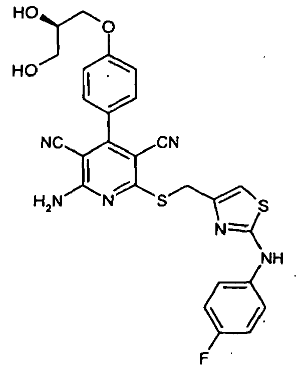 | 549 | 2.26 (4) | | 85 |
| **33** | 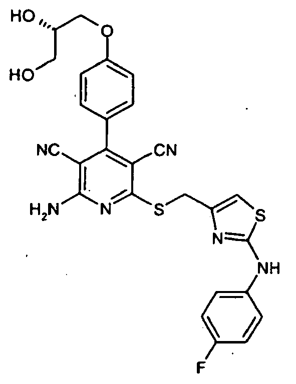 | 549 | 2.02 (3) | δ (400 MHz, DMSO-d₆) = 10.2 (br. s, 1H), 8.1 (br. s, 2H), 7.6-7-65 (m, 2H), 7.47 (d, 2H), 7.1-7.17 (m, 4H), 6.97 (s, 1H), 5.0 (br. s, 1H), 4.7 (br. s, 1H), 4.44 (s, 2H), 4.06-4.1 (m, 1H), 3.92-3.97 (m, 1H), 3.81 (m; 1H), 3.46 (dd, 2H). | 92 |
| **34** | 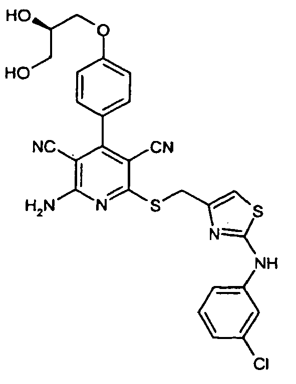 | 565 | 2.33 (2) | | 66 |
| **35** | 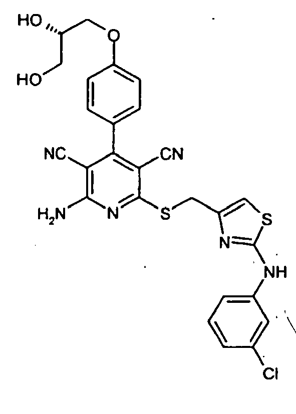 | 565 | 2.37 (4) | δ (400 MHz, DMSO-d₆) = 10.42 (s, 1H), 8.25-7.90 (br. s, 2H), 7.81 (s, 1H), 7.50-7.41 (m, 3H), 7.30 (t, 1H), 7.10 (d, 2H), 7.03 (s, 1H), 6.97 (s, 1H), 5.00 (d, 1H), 4.59 (t, 1H), 4.49 (s, 2H), 4.08 (dd, 1H), 3.95 (dd, 1H), 3.86-3.78 (m, 1H), 3.46 (t, 2H). | 80 |
| **36** | 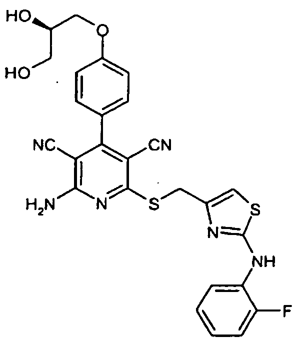 | 549 | 2.20 (2) | | 86 |
| **37** | 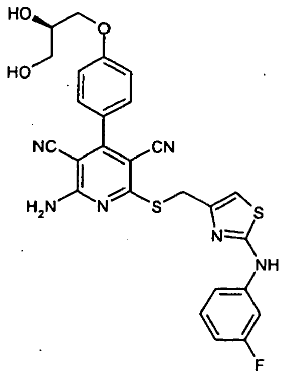 | 549 | 2.28 (4) | | 51 |
| **38** | 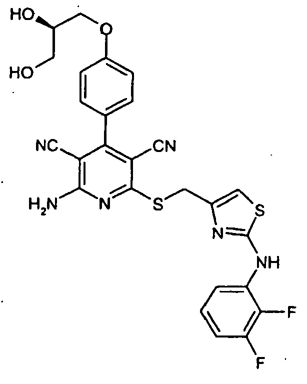 | 561 | 2.28 (4) | | 87 |
| **39** | 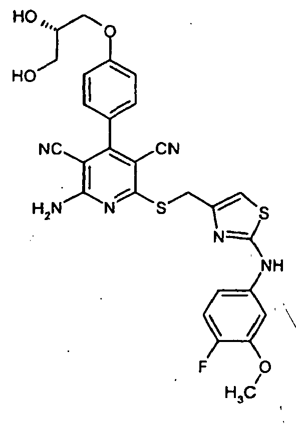 | 579 | 2.02 (3) | δ (400 MHz, DMSO-d₆)= 10.21 (s, 1H), 8.05 (br. s, 2H), 7.60 (dd, 1H), 7.47 (d, 2H), 7.14-7.00 (m, 4H), 6.97 (s, 1H), 4.99 (d, 1H), 4.68 (t, 1H), 4.47 (s; 2H), 4.09 (dd, 1H), 3.94 (dd, 1H), 3.86-3.78 (m, 1H), 3.80 (s, 3H), 3.46 (t, 2H). | 48 |
| **40** | 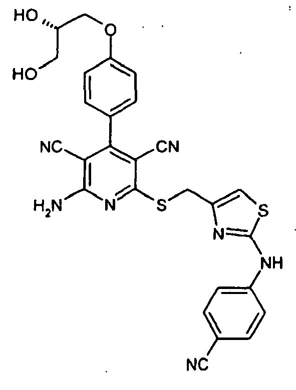 | 556 | 2.12 (2) | δ (400 MHz, DMSO-d₆) = 10.78 (s, 1H), 8.09 (br. s, 2H), 7.77 (dd, 4H), 7.48 (d, 2H), 7.14 (s, 1H), 7.10 (d, 2H), 4.99 (d, 1H), 4.69 (t, 1H), 4.50 (s, 2H), 4.08 (dd, 1H), 3.95 (dd, 1H), 3.86-3.77 (m, 1H), 3.47 (t, 2H). | 67 |
| **41** | 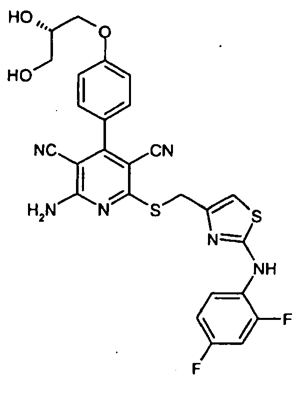 | 567 | 2.24 (2) | δ (400 MHz, DMSO-d₆) = 9.96 (s, 1H), 8.48-8.28 (m, 1H), 8.08 (br. s, 2H), 7.47 (dd, 2H), 7.34-7.26 (m, 1H), 7.09 (d, 2H), 7.05 (t, 1H), 6.99 (s, 1H), 4.99 (d, 1H), 4.69 (t, 1H), 4.43 (s, 2H), 4.08 (dd, 1H), 3.95 (dd, 1H), 3.86-3.78 (m, 1H), 3.46 (t, 2H). | 99 |
| **42** | 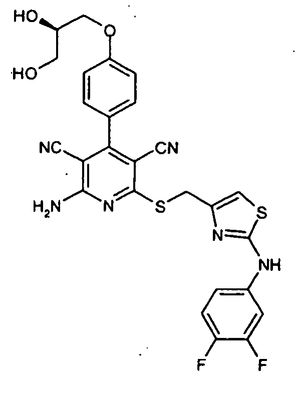 | 567 | 2.08 (3) | | 65 |
| **43** | 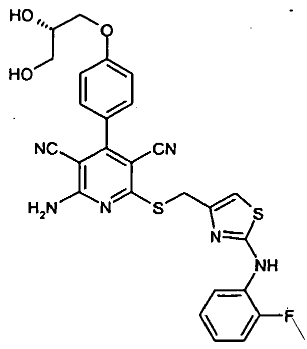 | 549 | 2.02 (3) | | 69 |
| **44** | 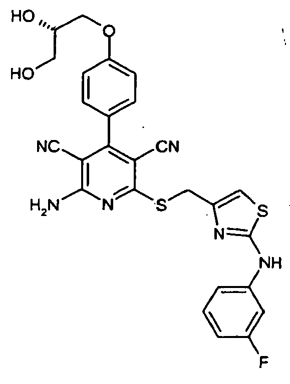 | 549 | 2.04 (3) | | 70 |
| **45** | 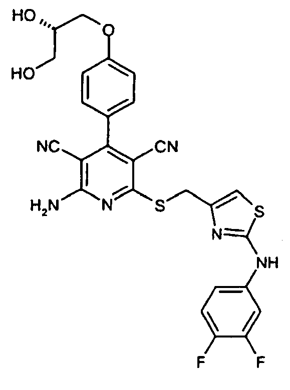 | 567 | 2.09 (3) | | 75 |
| **46** | 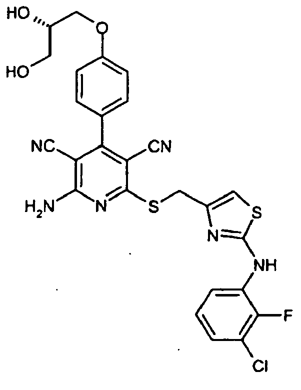 | 583 | 2.20 (3) | | 74 |
| **47** | 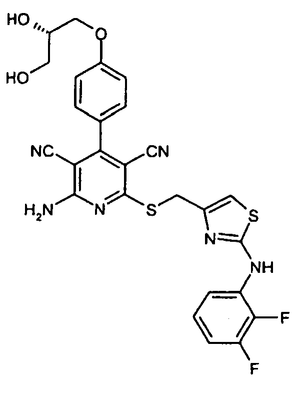 | 567 | 2.34 (4) | | 79 |
| **48** | 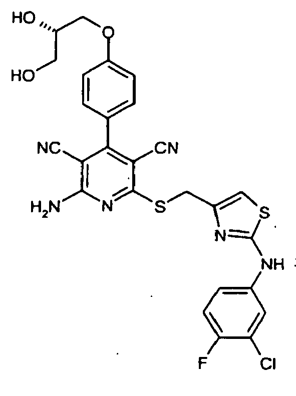 | 583 | 2.16 (3) | | 51 |
| **49** | 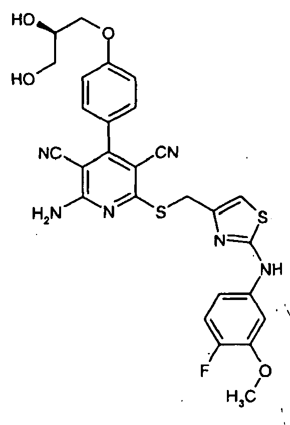 | 579 | 2.27 (4) | | 76 |
| **50** | 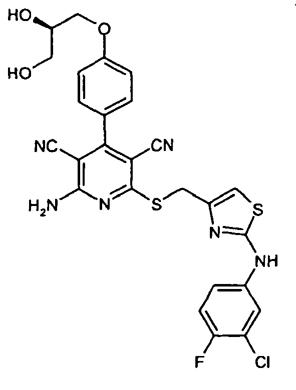 | 583 | 2.42 (4) | | 90 |
| **51** | 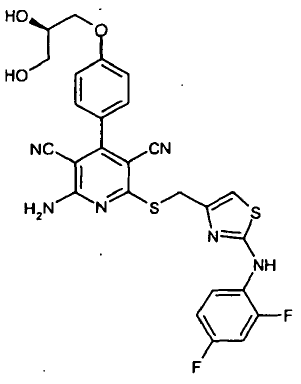 | 567 | 2.06 (3) | | 92 |
| **52** | 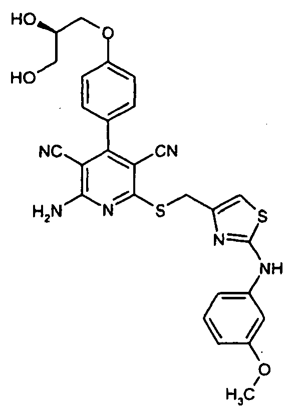 | 561 | 1.99 (3) | | 96 |
| **53** | 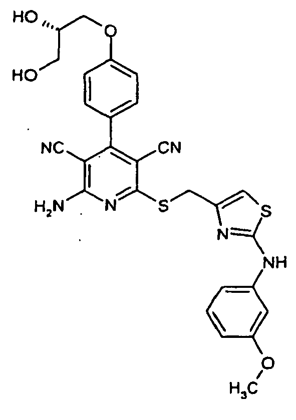 | 561 | 1.99 (3) | | 96 |
| **54** | 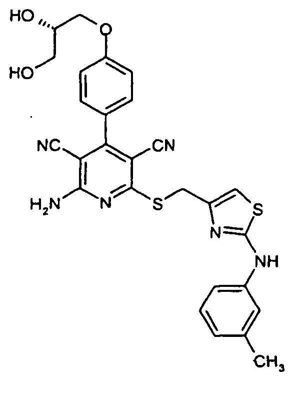 | 545 | 2.26 (2) | | 77 |
| **55** | 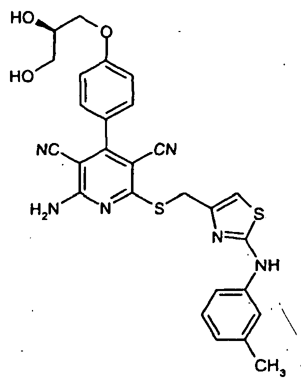 | 545 | 2.26 (2) | | 68 |
| **56** | 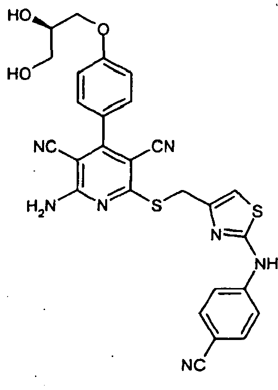 | 556 | 2.12 (2) | | 25 |

Analog zu Beispiel 16 werden die in Tabelle 6 aufgeführten Beispiele aus 2-Amino-4-[4-(2-hydroxyethoxy)phenyl]-6-mercaptopyridin-3,5-dicarbonitril hergestellt:

**Tabelle 6**

| **Beispiel Nr.** | **Struktur** | **MS (ESI) [M+H]⁺** | **LC-MS Rₜ [min] (Methode)** | **Ausbeute (% d. Th.)** |
|---|---|---|---|---|
| **57** | 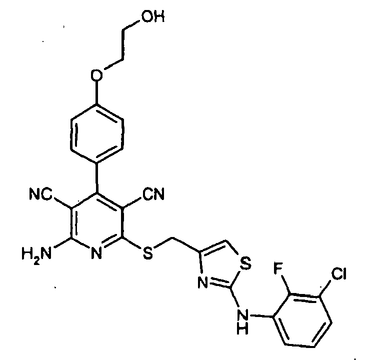 | 553 | 2.52 (2) | 68 |
| **58** | 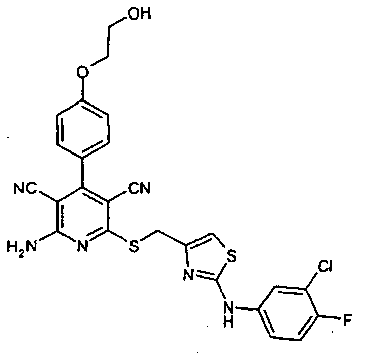 | 553 | 2.50 (2) | 42 |
| **59** | 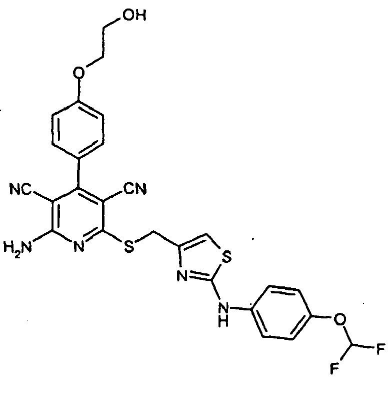 | 566 | 2.24 (3) | 20 |
| **60** | 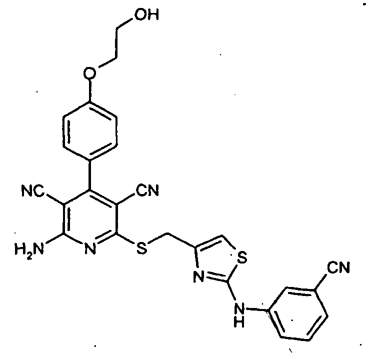 | 526 | 2.10 (3) | 83 |
| **61** | 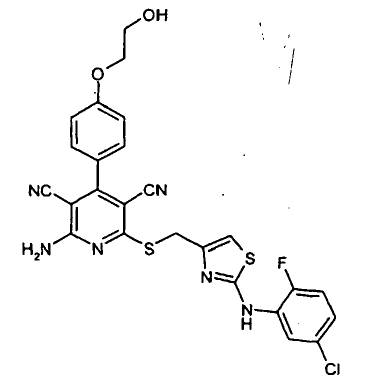 | 553 | 2.48 (2) | 70 |
| **62** | 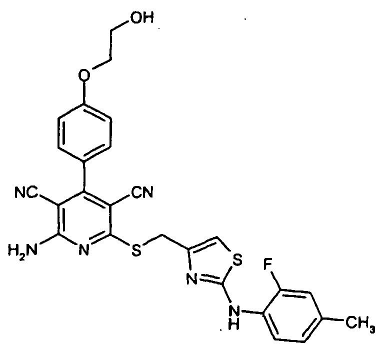 | 533 | 2.45 (2) | 70 |
| **63** | 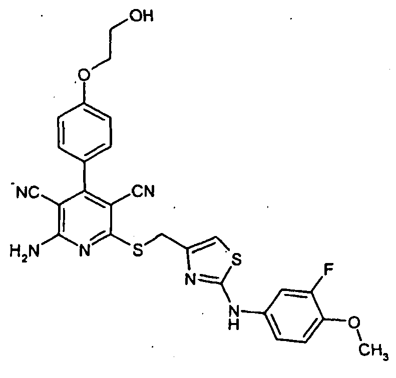 | 549 | 2.12 (3) | 66 |
| **64** | 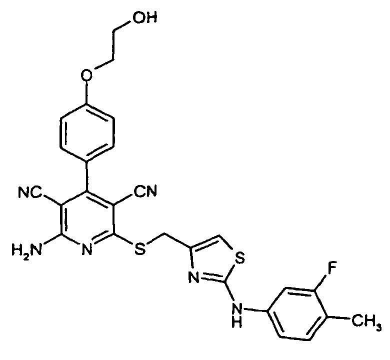 | 533 | 2.48 (2) | 54 |
| **65** | 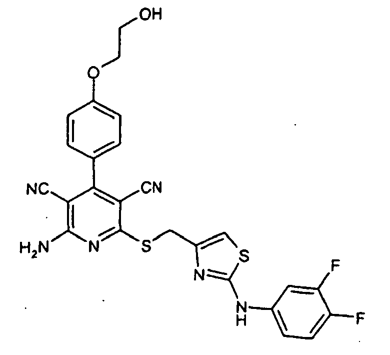 | 537 | 2.43 (2) | 34 |
| **66** | 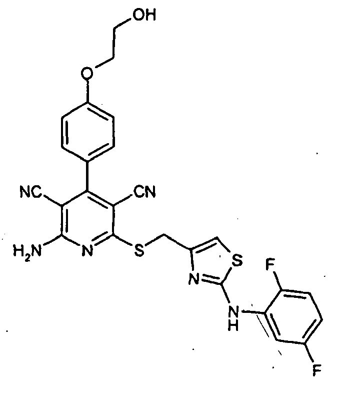 | 537 | 2.22 (3) | 16 |
| **67** | 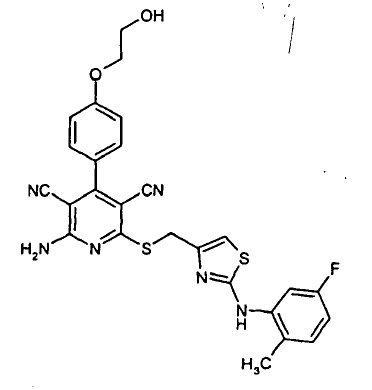 | 533 | 2.35 (2) | 20 |

### Beispiel 68

### 2-(2-Hydroxyethoxy)amino-6-[({2-[(4-fluorphenyl)amino]-1,3-thiazol-4-yl}methyl)thio]-4-[4-(2-hydroxyethoxy)phenyl]pyridin-3,5-dicarbonitril

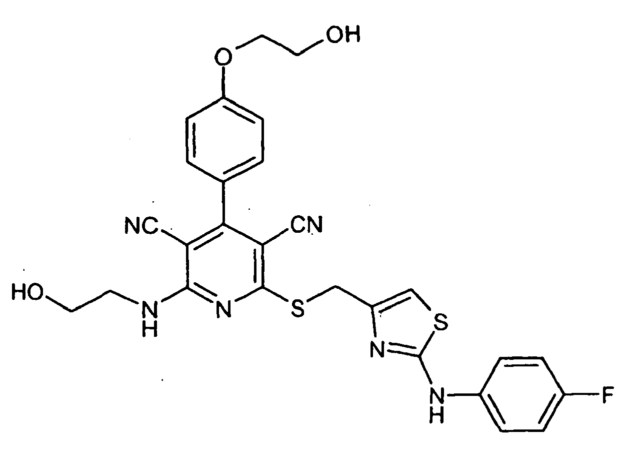

Analog zu Beispiel 8 erhält man die Titelverbindung durch Reaktion von 120 mg (0.70 mmol) 4-Fluorphenylthioharnstoff mit 89 mg (0.70 mmol) 1,3-Dichloraceton in Ethanol und anschliessende Umsetzung mit 245 mg (0.50 mmol) 2-(2-Hydroxyethoxy)amino-4-[4-(2-hydroxyethoxy)phenyl]-6-mercaptopyridin-3,5-dicarbonitril (Beispiel 32A).
Ausbeute: 30 mg (11% d. Th.)
¹H-NMR (400 MHz, DMSO-d₆): δ = 10.24 (s, 1H), 8.03 (t, 1H), 7.61 (dd, 2H), 7.46 (d, 2H), 7.12 (m, 4H), 6.81 (s, 1H), 4.91 (t, 1H), 4.80 (t, 1H), 4.50 (s, 2H), 4.07 (t, 2H), 3.74 (dt, 2H), 3.62 (t, 2H), 3.56 (m, 2H).
LC-MS (Methode 3): Rₜ = 2.10 min; MS (ESIpos): m/z = 563 [M+H]⁺.

### Beispiel 69

### 2-Amino-6-[({2-[(4-cyanophenyl)amino]-1,3-thiazol-4-yl}methyl)thio]-4- {4-[2-(dimethylamino)-ethoxy]phenyl}pyridin-3,5-dicarbonitril

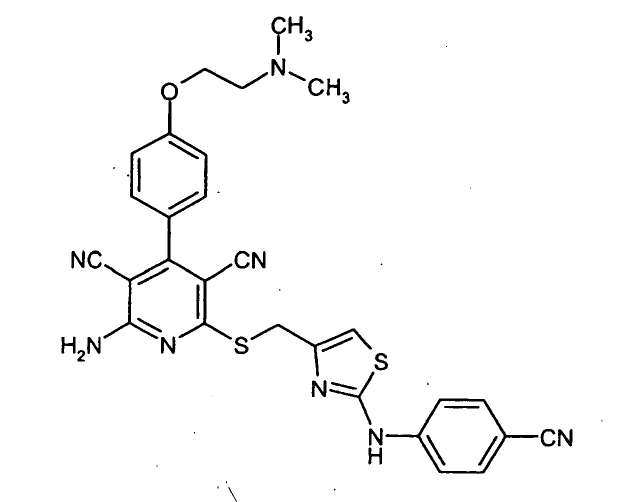

Eine Lösung von 26.6 mg (0.15 mmol) *N*-(4-Cyanophenyl)thioharnstoff and 19 mg (0.15 mmol) 1,3-Dichloraceton in 0.4 ml DMF wird 3 h lang bei +80°C gerührt. Nach Abkühlen auf RT wird eine Lösung von 50.9 mg (0.15 mmol) der Verbindung aus Beispiel 34A in 0.2 ml DMF sowie 50 mg (0.6 mmol) Natriumhydrogencarbonat zugegeben. Anschließend wird 12 h bei RT gerührt. Die Reaktionsmischung wird filtriert und direkt durch präparative HPLC gereinigt (Säule: Macherey Nagel VP50/21 Nucleosil 100-5 C18 Nautilus, 5 µm, 21 mm x 50 mm; Wellenlänge: 220 nm; Flussrate: 25 ml/min; Eluent A = Wasser + 0.1 % Ameisensäure, Eluent B = Acetonitril; Gradient: 0 min 10% B → 2 min 10% B → 6 min 90% B → 7 min 90% B → 7.1 min 10% B → 8 min 10% B) gereinigt. Die produkthaltigen Fraktionen werden vereinigt und am Rotationsverdampfer eingeengt.
Ausbeute: 50 mg (57% d. Th.)
¹H-NMR (400 MHz, DMSO-d₆): δ = 10.80 (s, 1H), 8.17 (s, 1H), 8.20-7.96 (br. s, 2H), 7.82-7.70 (m, 4H), 7.47 (d, 2H), 7.13 (d, 2H), 7.10 (s, 1H), 4.50 (s, 2H), 4.16 (t, 2H), 2.73 (t, 2H), 2.30 (s, 6H).
LC-MS (Methode 4): Rₜ = 1.87 min; MS (ESIpos): m/z = 553 [M+H]⁺.

### Beispiel 70

### 2-Amino-4-[4-(2-aminoethoxy)phenyl]-6-[({2-[(4-fluorphenyl)amino]-1,3-thiazol-4-yl}methyl)-thio]pyridin-3,5-dicarbonitril-Hydrochlorid

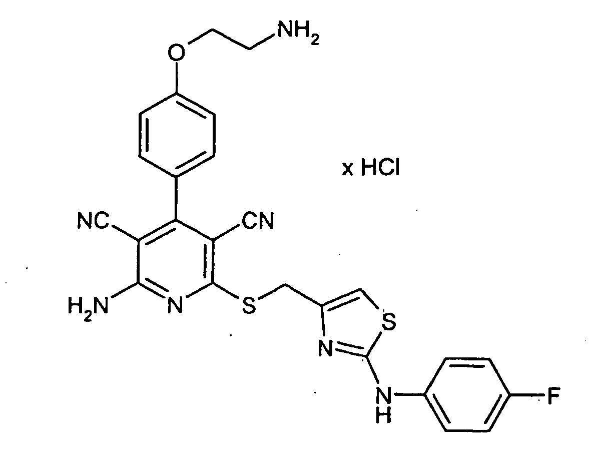

1000 mg (1.84 mmol) der Verbindung aus Beispiel 39A werden in 100 ml Dioxan gelöst, mit 150 mg (1.41 mmol) Palladium auf Aktivkohle versetzt und bei 3 bar mit Wasserstoff hydriert. Nach 3 h werden 4 ml 2 M Salzsäure zugegeben und für weitere 20 h bei 3 bar mit Wasserstoff hydriert. Der Ansatz wird dann über Seitz-Klärschichten-Filter abgesaugt, mit 50 ml Dioxan nachgewaschen und das Filtrat mit 50 ml Toluol versetzt. Nach Entfernen des Lösungsmittels am Rotationsverdampfer wird der Rückstand in einem Gemisch von 50 ml Wasser und 50 ml Essigsäureethylester aufgenommen. Der pH-Wert wird vorsichtig durch Zugabe von wässriger verdünnter Natriumhydrogencarbonat-Lösung auf ca. pH 9 eingestellt. Die gebildeten Phasen werden getrennt. Nach Trocknen der organischen Phase über Magnesiumsulfat wird das Lösungsmittel am Rotationsverdampfer entfernt und der Rückstand mittels präparativer HPLC gereinigt (Säule: YMC GEL ODS-AQ S-5 / 15 µm; Laufmittelgradient: Acetonitril/Wasser 10:90 → 95:5, unter Zusatz von 0.5% konzentrierter Salzsäure). Die produkthaltigen Fraktionen werden vereinigt und am Rotationsverdampfer eingeengt.
Ausbeute: 57 mg (6% d. Th.)
¹H-NMR (400 MHz, DMSO-d₆): δ = 10.27 (s, 1H), 8.08-7.97 (br. s, 2H), 7.67-7.59 (m, 2H), 7.51 (d, 2H), 7.20-7.09 (m, 4H), 6.98 (s, 1H), 4.47 (s, 2H), 4.25 (t, 2H), 3.31-3.21 (m, 2H).
LC-MS (Methode 2): Rₜ = 2.08 min; MS (ESIpos): m/z = 518 [M+H]⁺.

### Beispiel 71

### 2-Amino-6-[({2-[(4-fluorphenyl)amino]-1,3-thiazol-4-yl}methyl)thio]-4-[4-(2-hydroxypropoxy)-phenyl]pyridin-3,5-dicarbonitril

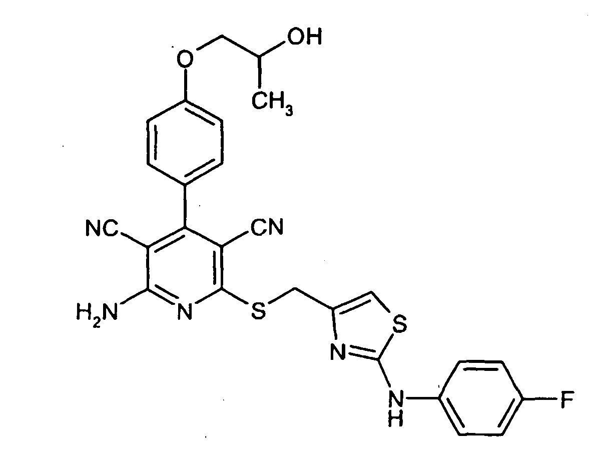

Eine Lösung von 43 mg (0.06 mmol) der Verbindung aus Beispiel 43A in 6 ml Methanol wird mit 3 ml 1 M Salzsäure versetzt und 20 h bei RT gerührt. Das Reaktionsgemisch wird daraufhin mit 10 ml gesättigter Natriumhydrogencarbonat-Lösung versetzt und mit Essigsäureethylester (dreimal je 10 ml) extrahiert. Die vereinigten organischen Phasen werden über Magnesiumsulfat getrocknet. Nach Entfernen des Lösungsmittels am Rotationsverdampfer wird das Rohprodukt an Kieselgel 60 (Laufmittelgradient Dichlormethan/Ethanol 100:1 → 20:1) chromatographisch gereinigt.
Ausbeute: 0.34 g (96% d. Th.)
¹H-NMR (400 MHz, DMSO-d₆): δ = 10.22 (s, 1H), 8.22-7.91 (br. s, 2H), 7.61 (dd, 2H), 7.47 (d, 2H), 7.18-7.06 (m, 4H), 6.97 (s, 1H), 4.91 (d, 1H), 4.46 (s, 2H), 4.02-3.94 (m, 1H), 3.94-3.83 (m, 2H), 1.17 (d, 3H).
LC-MS (Methode 3): Rₜ = 2.26 min; MS (ESIpos): m/z = 533 [M+H]⁺.

### B. Bewertung der pharmakologischen und physiologischen Wirksamkeit

Die pharmakologische und physiologische Wirkung der erfindungsgemäßen Verbindungen kann in folgenden Assays gezeigt werden:
B-1. Indirekte Bestimmung des Adenosin-Agonismus über Genexpression
Zellen der permanenten Linie CHO (Chinese Hamster Ovary) werden stabil mit der cDNA für die Adenosin-Rezeptor-Subtypen A1, A2a und A2b transfiziert. Die Adenosin-A1-Rezeptoren sind über Gᵢ-Proteine und die Adenosin-A2a- und A2b-Rezeptoren über Gₛ-Proteine an die Adenylatcyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luziferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration und Medium-Zusammensetzung. Zur pharmakologischen Charakterisierung der Zellen und zum Roboter-gestützten Substanz-Screening wird das folgende Testprotokoll verwendet:
Die Stammkulturen werden in DMEM/F12-Medium mit 10% FCS (fötales Kälberserum) bei 37°C unter 5% CO₂ gezüchtet und jeweils nach 2-3 Tagen 1:10 gesplittet. Testkulturen werden mit 2000 Zellen pro Napf in 384-well-Platten ausgesät und ca. 48 Stunden bei 37°C angezogen. Dann wird das Medium durch eine physiologische Kochsalzlösung (130 mM Natriumchlorid, 5 mM Kaliumchlorid, 2 mM Calciumchlorid, 20 mM HEPES, 1 mM Magnesiumchlorid-Hexahydrat, 5 mM Natriumhydrogencarbonat, pH 7.4) ersetzt. Die in DMSO gelösten zu testenden Substanzen werden in einer Verdünnungsreihe von 1.1 x 10⁻¹¹ M bis 3 x 10⁻⁶ M (Endkonzentration) zu den Testkulturen pipettiert (maximale DMSO-Endkonzentration im Testansatz: 0.5%). 10 Minuten später wird Forskolin zu den A1-Zellen zugegeben und anschließend werden alle Kulturen für vier Stunden bei 37°C inkubiert. Danach wird zu den Testkulturen 35 µl einer Lösung, bestehend zu 50% aus Lysereagenz (30 mM Dinatriumhydrogenphosphat, 10% Glycerin, 3% TritonX100, 25 mM TrisHCl, 2 mM Dithiotreitol (DTT), pH 7.8) und zu 50% aus Luciferase-Substrat-Lösung (2.5 mM ATP, 0.5 mM Luciferin, 0.1 mM Coenzym A, 10 mM Tricin, 1.35 mM Magnesiumsulfat, 15 mM DTT, pH 7.8) zugegeben, ca. 1 Minute geschüttelt und die Luciferase-Aktivität mit einem Kamerasystem gemessen. Bestimmt werden die EC₅₀-Werte, d.h. die Konzentrationen, bei denen bei der A1-Zelle 50% der Luciferase-Antwort inhibiert bzw. bei den A2b- und A2a-Zellen 50% der maximalen Stimulierbarkeit mit der entsprechenden Substanz erreicht sind. Als Referenzverbindung dient in diesen Experimenten die Adenosin-analoge Verbindung NECA (5-N-Ethylcarboxamido-adenosin), die mit hoher Affinität an alle Adenosin-Rezeptor-Subtypen bindet und eine agonistische Wirkung besitzt [Klotz, K.N., Hessling, J., Hegler, J., Owman, C., Kull, B., Fredholm, B.B., Lohse, M.J., "Comparative pharmacology of human adenosine receptor subtypes - characterization of stably transfected receptors in CHO cells", Naunyn Schmiedebergs Arch. Pharmacol. 357, 1-9 (1998)].

In der folgenden Tabelle 1 sind die EC₅₀-Werte repräsentativer Ausführungsbeispiele für die Rezeptorstimulation an Adenosin A1-, A2a- und A2b-Rezeptor-Subtypen aufgeführt:

**Tabelle 1**

| **Beispiel Nr.** | **EC₅₀ A1 [nM]** | **EC₅₀ A2a** | **EC₅₀ A2b** |
|---|---|---|---|
| | **(1 µM Forskolin)** | **[nM]** | **[nM]** |
| **2** | 9.9 | 747 | 6.1 |
| **8** | 1 | 300 | 1 |
| **10** | 2 | >200 | 1 |
| **13** | 0.2 | 236 | 0.1 |
| **21** | 0.7 | 103 | 0.5 |
| **26** | 23 | >3000 | 74 |
| **35** | 0.4 | 142 | 0.3 |
| **39** | 0.3 | 1200 | 1.4 |
| **44** | 0.6 | 140 | 0.3 |
| **48** | 0.4 | 140 | 0.1 |
| **70** | 2.3 | >3000 | 28 |

B-2. Untersuchung an isolierten Gefäßen
Aus narkotisierten Ratten wird die Arteria caudalis präpariert und in eine konventionelle Apparatur zur Messung isolierter Gefäße eingespannt. Die Gefäße werden in einem Wärmebad perfundiert und mit Phenylephrin kontrahiert. Das Maß der Kontraktion wird über einen Kontraktionsmesser ermittelt. Zu den vorkontrahierten Gefäßen werden Testsubstanzen gegeben und die Abnahme der Kontraktion der Gefäße gemessen. Eine Abnahme der Kontraktion entspricht einer Dilatation der Gefäße. Als EC₅₀-Wert einer Testsubstanz bzgl. ihrer relaxierenden Eigenschaften wird die Konzentration angegeben, bei der die Kontraktion der Gefäße um 50% verringert ist.
B-3. Langendorff-Herz-Untersuchungen
Narkotisierten Ratten wird nach Eröffnung des Brustkorbes das Herz schnell entnommen und in eine konventionelle Langendorff-Apparatur eingeführt. Die Koronararterien werden volumenkonstant (10 ml/min) perfundiert und der dabei auftretende Perfusionsdruck wird über einen entsprechenden Druckaufnehmer registriert. Eine Abnahme des Perfusionsdrucks in dieser Anordnung entspricht einer Relaxation der Koronararterien. Gleichzeitig wird über einen in die linke Herzkammer eingeführten Ballon und einen weiteren Druckaufnehmer der Druck gemessen, der vom Herzen während jeder Kontraktion entwickelt wird. Die Frequenz des isoliert schlagenden Herzens wird rechnerisch aus der Anzahl der Kontraktionen pro Zeiteinheit ermittelt.
B-4. Blutdruck- und Herzfrequenz-Messungen an wachen Ratten
Wachen SHR (spontaneously hypertensive rats)-Ratten, die einen internen Sender tragen, der dauerhaft sowohl Blutdruck als auch Herzfrequenz messen kann (telemetrische Erfassung von hämodynamischen Parametern), werden Testsubstanzen in verschiedenen Dosierungen oral verabreicht. Anschließend wird über 24 Stunden Blutdruck und Herzfrequenz und deren Veränderung aufgezeichnet.
B-5. Blutdruck- und Herzfrequenz-Messungen am wachen Rhesus- und Krallenaffen
Wache Rhesusaffen werden in einer Röhre fixiert. Den Tieren werden Katheder zur Infusion von Testsubstanzen und zur Blutabnahme in die Beinvenen eingesetzt. Testsubstanzen werden in unterschiedlichen Konzentrationen über 15-30 Minuten durch einen der Katheder intravenös infundiert. Veränderungen des Blutdrucks und der Pulsfrequenz werden über ein handelsübliches Frühgeborenen-Blutdruckmessgerät alle 1-5 Minuten über insgesamt 60 Minuten erfasst. Die Messmanschette wird dazu an einem der Beine befestigt.
Wachen Krallenaffen, die einen internen Sender tragen, der dauerhaft sowohl Blutdruck als auch Herzfrequenz messen kann (telemetrische Erfassung von hämodynamischen Parametern), werden Testsubstanzen in verschiedenen Konzentrationen oral verabreicht. Anschließend wird über 6-24 Stunden Blutdruck und Herzfrequenz und deren Veränderungen aufgezeichnet.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluss der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 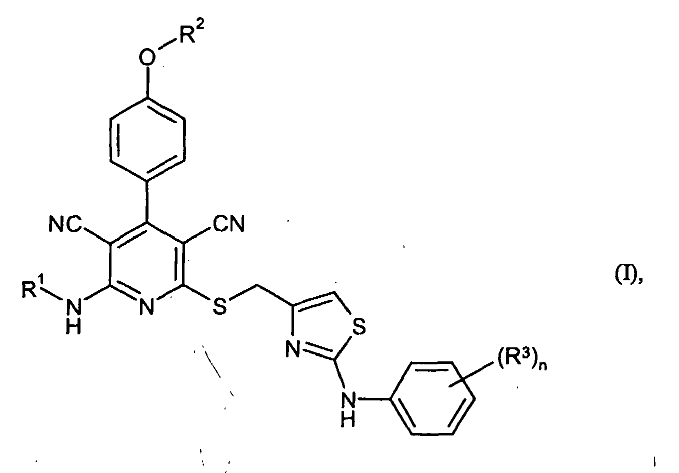 in welcher
R¹ für Wasserstoff oder für (C₁-C₆)-Alkyl steht, das durch Hydroxy, Amino, Mono-oder Di-(C₁-C₄)-alkylamino, Pyrrolidino, Piperidino, Morpholino, Piperazino oder *N'*-Methylpiperazino substituiert sein kann,
R² für (C₂-C₆)-Alkyl steht, das ein- oder zweifach, gleich oder verschieden, mit Substituenten ausgewählt aus der Gruppe von Hydroxy, (C₁-C₄)-Alkoxy, Amino, Mono- und Di-(C₁-C₄)-alkylamino substituiert ist,
R³ für einen Substituenten ausgewählt aus der Gruppe von Halogen, Cyano, Nitro, (C₁-C₆)-Alkyl, Hydroxy, (C₁-C₆)-Alkoxy, Amino, Mono- und Di-(C₁-C₆)-alkylamino, Carboxyl und (C₁-C₆)-Alkoxycarbonyl steht,
worin Alkyl und Alkoxy ihrerseits jeweils bis zu fünffach mit Fluor substituiert sein können,
und
n für die Zahl 0, 1, 2, 3, 4 oder 5 steht,
wobei für den Fall, dass der Substituent R³ mehrfach auftritt, seine Bedeutungen gleich oder verschieden sein können,
sowie ihre Salze, Solvate und Solvate der Salze.

2. Verbindung der Formel (I) nach Anspruch 1,
in welcher
R¹ für Wasserstoff oder für (C₁-C₄)-Alkyl steht, das mit Hydroxy, Amino oder Dimethylamino substituiert sein kann,
R² für (C₂-C₄)-Alkyl steht, das ein- oder zweifach, gleich oder verschieden, mit Substituenten ausgewählt aus der Gruppe von Hydroxy, Methoxy und Amino substituiert ist,
R³ für einen Substituenten ausgewählt aus der Gruppe von Halogen, Cyano, Nitro, (C₁-C₄)-Alkyl, Hydroxy, (C₁-C₄)-Alkoxy, Amino, Mono- und Di-(C₁-C₄)-alkylamino, Carboxyl und (C₁-C₄)-Alkoxycarbonyl steht,
worin Alkyl und Alkoxy ihrerseits jeweils bis zu dreifach mit Fluor substituiert sein können,
und
n für die Zahl 0, 1 oder 2 steht,
wobei für den Fall, dass der Substituent R³ zweifach auftritt, seine Bedeutungen gleich oder verschieden sein können,
sowie ihre Salze, Solvate und Solvate der Salze.

3. Verbindung der Formel (I) nach Anspruch 1 oder 2,
in welcher
R¹ für Wasserstoff steht,
R² für Ethyl, n-Propyl oder iso-Propyl steht, die jeweils ein- oder zweifach, gleich oder verschieden, mit Substituenten ausgewählt aus der Gruppe von Hydroxy, Methoxy und Amino substituiert sind,
R³ für einen Substituenten ausgewählt aus der Gruppe von Fluor, Chlor, Brom, Cyano, Nitro, Methyl, Ethyl, Trifluormethyl, Hydroxy, Methoxy, Ethoxy, Amino, Mono- und Dimethylamino, Carboxyl, Methoxycarbonyl und Ethoxycarbonyl steht,
und
n für die Zahl 0, 1 oder 2 steht,
wobei für den Fall, dass der Substituent R³ zweifach auftritt, seine Bedeutungen gleich oder verschieden sein können,
sowie ihre Salze, Solvate und Solvate der Salze.

4. Verfahren zur Herstellung einer Verbindung der Formel (I), wie in den Ansprüchen 1 bis 3 definiert, **dadurch gekennzeichnet, dass** man eine Verbindung der Formel (II) 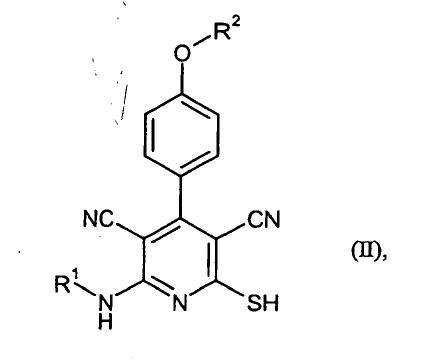 worin R¹ und R² jeweils die in den Ansprüchen 1 bis 3 angegebenen Bedeutungen haben,
mit einer Verbindung der Formel (III) 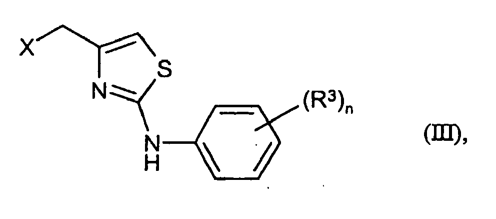 worin R³ und n jeweils die in den Ansprüchen 1 bis 3 angegebenen Bedeutungen haben und
X für eine geeignete Abgangsgruppe steht
umsetzt
und die so erhaltenen Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

5. Verbindung, wie in einem der Ansprüche 1 bis 3 definiert, zur Behandlung und/oder Prophylaxe von Krankheiten.

6. Verwendung einer Verbindung, wie in einem der Ansprüche 1 bis 3 definiert, zur Herstellung eines Arzneimittels zur Behandlung und/oder Prävention von Hypertonie und anderen Erkrankungen des Herzkreislauf-Systems.

7. Arzneimittel enthaltend eine Verbindung, wie in einem der Ansprüche 1 bis 3 definiert, in Kombination mit einem inerten, nicht-toxischen, pharmazeutisch geeigneten Hilfsstoff.

8. Arzneimittel enthaltend eine Verbindung, wie in einem der Ansprüche 1 bis 3 definiert, in Kombination mit einem weiteren Wirkstoff ausgewählt aus der Gruppe bestehend aus Betablocker, Calcium-Antagonisten, Diuretika, ACE-Hemmer, AT1-Antagonisten und Nitrate.

9. Arzneimittel nach Anspruch 7 oder 8 zur Behandlung und/oder Prävention von Hypertonie und anderen Erkrankungen des Herzkreislauf-Systems.

## Claims

1. Compound of the formula (I) 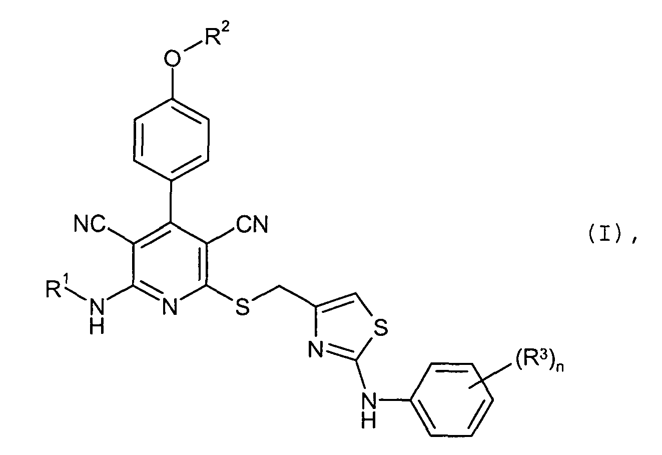 in which
R¹ represents hydrogen or represents (C₁-C₆)-alkyl which may be substituted by hydroxyl, amino, mono- or di-(C₁-C₄)-alkylamino, pyrrolidino, piperidino, morpholino, piperazino or *N'-*methylpiperazino,
R² represents (C₂-C₆)-alkyl which is mono- or disubstituted by identical or different substituents selected from the group consisting of hydroxyl, (C₁-C₄)-alkoxy, amino, mono- and di-(C₁-C₄)-alkylamino,
R³ represents a substituent selected from the group consisting of halogen, cyano, nitro, (C₁-C₆)-alkyl, hydroxyl, (C₁-C₆)-alkoxy, amino, mono- and di-(C₁-C₆)-alkylamino, carboxyl and (C₁-C₆)-alkoxycarbonyl,
where alkyl and alkoxy for their part may in each case be substituted up to five times by fluorine,
and
n represents the number 0, 1, 2, 3, 4 or 5,
where, if the substituent R³ is present more than once, its meanings may be identical or different,
and its salts, solvates and solvates of the salts.

2. Compound of the formula (I) according to Claim 1,
in which
R¹ represents hydrogen or represents (C₁-C₄)-alkyl which may be substituted by hydroxyl, amino or dimethylamino,
R² represents (C₂-C₄)-alkyl which is mono- or disubstituted by identical or different substituents selected from the group consisting of hydroxyl, methoxy and amino,
R³ represents a substituent selected from the group consisting of halogen, cyano, nitro, (C₁-C₄)-alkyl, hydroxyl, (C₁-C₄)-alkoxy, amino, mono- and di-(C₁-C₄)-alkylamino, carboxyl and (C₁-C₄)-alkoxycarbonyl,
where alkyl and alkoxy for their part may in each case be substituted up to three times by fluorine,
and
n represents the number 0, 1 or 2,
where, if the substituent R³ is present twice, its meanings may be identical or different,
and its salts, solvates and solvates of the salts.

3. Compound of the formula (I) according to Claim 1 or 2,
in which
R¹ represents hydrogen,
R² represents ethyl, n-propyl or isopropyl which are in each case mono- or disubstituted by identical or different substituents selected from the group consisting of hydroxyl, methoxy and amino,
R³ represents a substituent selected from the group consisting of fluorine, chlorine, bromine, cyano, nitro, methyl, ethyl, trifluoromethyl, hydroxyl, methoxy, ethoxy, amino, mono- and dimethylamino, carboxyl, methoxycarbonyl and ethoxycarbonyl,
and
n represents the number 0, 1 or 2,
where, if the substituent R³ is present twice, its meanings may be identical or different,
and its salts, solvates and solvates of the salts.

4. Process for preparing a compound of the formula (I) as defined in Claims 1 to 3, **characterized in that** a compound of the formula (II) 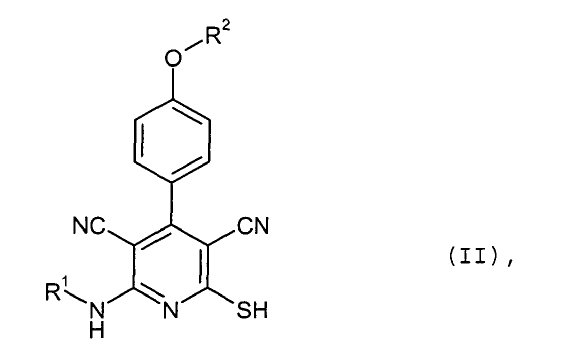 in which R¹ and R² each have the meanings given in Claims 1 to 3,
is reacted with a compound of the formula (III) 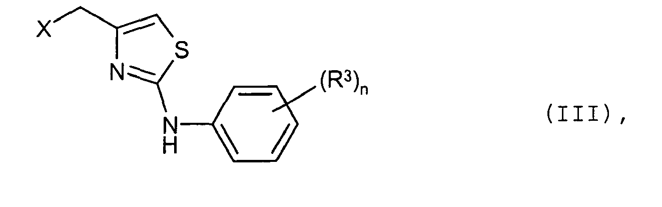 in which R³ and n each have the meanings given in Claims 1 to 3 and
X represents a suitable leaving group, and the resulting compounds of the formula (I) are, if appropriate, converted into their solvates, salts and/or solvates of the salts using the appropriate (i) solvents and/or (ii) bases or acids.

5. Compound as defined in any of Claims 1 to 3 for the treatment and/or prophylaxis of diseases.

6. Use of a compound as defined in any of Claims 1 to 3 for preparing a medicament for the treatment and/or prevention of hypertension and other disorders of the cardiovascular system.

7. Medicament, comprising a compound as defined in any of Claims 1 to 3 in combination with an inert, nontoxic, pharmaceutically suitable auxiliary.

8. Medicament, comprising a compound as defined in any of Claims 1 to 3 in combination with a further active compound selected from the group consisting of beta blockers, calcium antagonists, diuretics, ACE inhibitors, AT1 antagonists and nitrates.

9. Medicament according to Claim 7 or 8 for the treatment and/or prevention of hypertension and other disorders of the cardiovascular system.

## Revendications

1. Composé de formule (I) 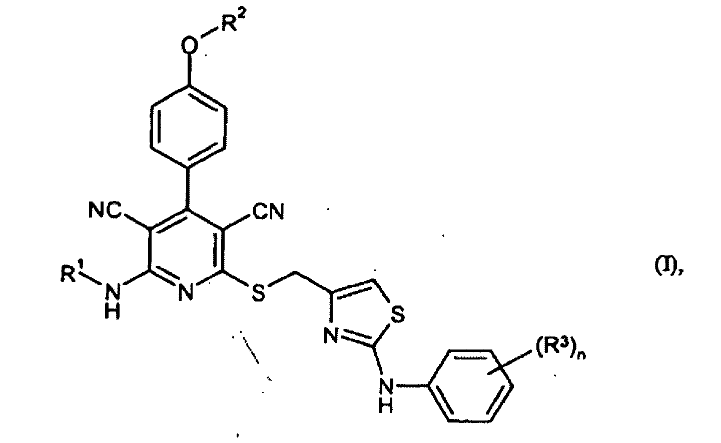 dans laquelle
R¹ représente l'hydrogène ou un groupe alkyle en C₁ à C₆, qui peut être substitué par un reste hydroxy, amino, mono- ou di-(alkyle en C₁ à C₄)amino, pyrrolidino, pipéridino, morpholino, pipérazino ou *N'*-méthylpipérazino,
R² représente un groupe alkyle en C₂ à C₆, qui est substitué une ou deux fois identiques ou différentes avec des substituants choisis dans le groupe des restes hydroxy, alkoxy en C₁ à C₄, amino, mono- et di-(alkyle en C₁ à C₄)amino,
R³ représente un substituant choisi dans le groupe des restes halogéno, cyano, nitro, alkyle en C₁ à C₆, hydroxy, alkoxy en C₁ à C₆, amino, mono- et di-(alkyle en C₁ à C₆)amino, carboxyle et (alkoxy en C₁ à C₆)carbonyle,
les restes alkyle et alkoxy pouvant eux-mêmes être substitués dans chaque cas jusqu'à cinq fois avec du fluor,
et
n représente le nombre 0, 1, 2, 3, 4 ou 5, le substituant R³, au cas où il apparaît plusieurs fois, pouvant avoir des définitions identiques ou différentes,
ainsi que ses sels, ses produits de solvatation et les produits de solvatation de ses sels.

2. Composé de formule (I) suivant la revendication 1, dans lequel
R¹ représente l'hydrogène ou un groupe alkyle en C₁ à C₄, qui peut être substitué par un reste hydroxy, amino ou diméthylamino,
R² représente un reste alkyle en C₂ à C₄, qui est substitué une ou deux fois identiques ou différentes avec des substituants choisis dans le groupe des restes hydroxy, méthoxy et amino,
R³ représente un substituant choisi dans le groupe des restes halogéno, cyano, nitro, alkyle en C₁ à C₄, hydroxy, alkoxy en C₁ à C₄, amino, mono- et di-(alkyle en C₁ à C₄)amino, carboxyle et (alkoxy en C₁ à C₄)-carbonyle, les restes alkyle et alkoxy pouvant eux-mêmes être substitués dans chaque cas jusqu'à trois fois avec du fluor,
et
n représente le nombre 0, 1 ou 2, le substituant R³, au cas où il apparaît deux fois, pouvant avoir des définitions identiques ou différentes,
ainsi que ses sels, ses produits de solvatation et les produits de solvatation de ses sels.

3. Composé de formule (I) suivant la revendication 1 ou 2,
dans lequel,
R¹ représente l'hydrogène,
R² représente un reste éthyle, n-propyle ou isopropyle qui sont substitués dans chaque cas une ou deux fois identiques ou différentes avec des substituants choisis dans le groupe des restes hydroxy, méthoxy et amino,
R³ représente un substituant choisi dans le groupe des restes fluoro, chloro, bromo, cyano, nitro, méthyle, éthyle, trifluorométhyle, hydroxy, méthoxy, éthoxy, amino, mono- et diméthylamino, carboxyle, méthoxycarbonyle et éthoxycarbonyle,
et
n représente le nombre 0, 1 ou 2,
le substituant R³, au cas où il apparaît deux fois, pouvant avoir des définitions identiques ou différentes,
ainsi que ses sels, ses produits de solvatation et les produits de solvatation de ses sels.

4. Procédé de production d'un composé de formule (I), tel que défini dans les revendications 1 à 3, **caractérisé en ce qu'**on fait réagir un composé de formule (II) 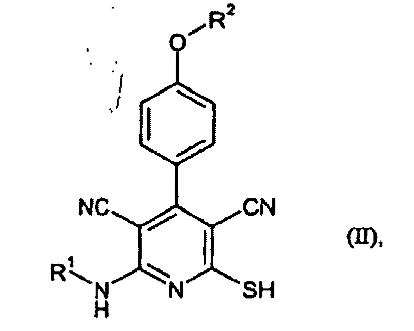 dans laquelle R¹ et R² ont dans chaque cas les définitions indiquées dans les revendications 1 à 3,
avec un composé de formule (III) 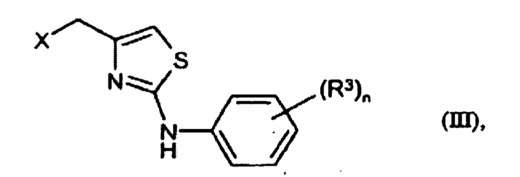 dans laquelle R³ et n ont chacun les définitions indiquées dans les revendications 1 à 3,
et
X représente un groupe partant convenable,
et on fait éventuellement réagir les composés de formule (I) ainsi obtenus avec (i) les solvants correspondants et/ou (ii) les bases correspondantes ou les acides correspondants, pour obtenir ses produits de solvatation, ses sels et/ou les produits de solvatation de ses sels.

5. Composé tel que défini dans l'une des revendications 1 à 3, destiné au traitement et/ou à la prophylaxie de maladies.

6. Utilisation d'un composé tel que défini dans l'une des revendications 1 à 3 pour la préparation d'un médicament destiné au traitement et/ou à la prévention de l'hypertonie et d'autres maladies du système cardio-vasculaire.

7. Médicament contenant un composé tel que défini dans l'une des revendications 1 à 3, en combinaison avec une substance pharmaceutique appropriée inerte, non toxique.

8. Médicament contenant un composé tel que défini dans l'une des revendications 1 à 3, en combinaison avec une autre substance active choisie dans le groupe constitué de β-bloquants, d'antagonistes du calcium, de diurétiques, d'inhibiteurs de l'enzyme de conversion de l'angiotensine, d'antagonistes d'AT1, et de nitrates.

9. Médicament suivant la revendication 7 ou 8, destiné au traitement et/ou à la prévention de l'hypertonie et d'autres maladies du système cardio-vasculaire.
